# EUROPEAN PATENT APPLICATION

(11) **EP 1 340 979 A2**
(43) Date of publication of application: **03.09.2003**
(21) Application number: 03250902.8
(22) Date of filing: 13.02.2003
(51) Int. Cl.: G01N 33/74, C12N 15/00, C12N 15/10, C07K 14/72, A61K 38/00

(54) **Neuropeptide receptor and uses thereof**

(30) Priority: 27.02.2002 GB 0204610; 11.10.2002 GB 0223720
(71) Applicant: Pfizer Limited, Sandwich, Kent CT13 9NJ (GB); PFIZER INC., New York, N.Y. 10017 (US)
(72) Inventor: Fidock, Mark David, c/o Pfizer Global Res. & Dev., Sandwich, Kent CT13 9NJ (GB); Robas, Nicola M., c/o Pfizer Global Res. & Dev., Sandwich, Kent CT13 9NJ (GB)
(74) Representative: Hayles, James Richard

(57) **Abstract**

This invention relates to the identification of an orphan G-protein coupled receptor MRGX2 (and variants thereof) as a receptor of neuropeptides such as but not limited to cortistatin, somatostatin, Bam 13-22, α-MSH, neuropeptide FF, dynorphin A and substance P, and the use of such neuropeptides (and structurally related molecules) as modulators for MRGX2. It also relates to screening methods to identify agonists and antagonists for this neuropeptide receptor.

SEQUENCE LISTING

<110> Pfizer Ltd. (EP(GB) only)
      Pfizer Inc. (JP, US, EP except GB)

<120> Neuropeptide receptor and uses thereof

<130> PC22069A

<150> GB 0204610.0
<151> 2002-02-27

<150> GB 0223720.4
<151> 2002-10-11

<160> 6

<170> FastSEQ for Windows Version 4.0
<210> 1
<211> 993
<212> DNA
<213> Homo sapiens

<400> 1
atggatccaa ccaccccggc ctggggaaca gaaagtacaa cagtgaatgg aaatgaccaa 60
gcccttcttc tgctttgtgg caaggagacc ctgatcccgg tcttcctgat ccttttcatt 120
gccctggtcg ggctggtagg aaacgggttt gtgctctggc tcctgggctt ccgcatgcgc 180
aggaacgcct tctctgtcta cgtcctcagc ctggccgggg ccgacttcct cttcctctgc 240
ttccagatta taaattgcct ggtgtacctc agtaacttct tctgttccat ctccatcaat 300
ttccctagct tcttcaccac tgtgatgacc tgtgcctacc ttgcaggcct gagcatgctg 360
agcaccgtca gcaccgagcg ctgcctgtcc gtcctgtggc ccatctggta tcgctgccgc 420
cgccccagac acctgtcagc ggtcgtgtgt gtcctgctct gggccctgtc cctactgctg 480
agcatcttgg aagggaagtt ctgtggcttc ttatttagtg atggtgactc tggttggtgt 540
cagacatttg atttcatcac tgcagcgtgg ctgatttttt tattcatggt tctctgtggg 600
tccagtctgg ccctgctggt caggatcctc tgtggctcca ggggtctgcc actgaccagg 660
ctgtacctga ccatcctgct cacagtgctg gtgttcctcc tctgcggcct gccctttggc 720
attcagtggt tcctaatatt atggatctgg aaggattctg atgtcttatt ttgtcatatt 780
catccagttt cagttgtcct gtcatctctt aacagcagtg ccaaccccat catttacttc 840
ttcgtgggct cttttaggaa gcagtggcgg ctgcagcagc cgatcctcaa gctggctctc 900
cagagggctc tgcaggacat tgctgaggtg gatcacagtg aaggatgctt ccgtcagggc 960
accccggaga tgtcgagaag cagtctggtg tag 993

<211> 330
<212> PRT
<213> Homo sapiens

<400> 2
Met Asp Pro Thr Thr Pro Ala Trp Gly Thr Glu Ser Thr Thr Val Asn
1               5                   10                  15
Gly Asn Asp Gln Ala Leu Leu Leu Leu Cys Gly Lys Glu Thr Leu Ile
            20                  25                  30
Pro Val Phe Leu Ile Leu Phe Ile Ala Leu Val Gly Leu Val Gly Asn
        35                  40                  45
Gly Phe Val Leu Trp Leu Leu Gly Phe Arg Met Arg Arg Asn Ala Phe
    50                  55                  60
Ser Val Tyr Val Leu Ser Leu Ala Gly Ala Asp Phe Leu Phe Leu Cys
65                  70                  75                  80
Phe Gln Ile Ile Asn Cys Leu Val Tyr Leu Ser Asn Phe Phe Cys Ser
                85                  90                  95
Ile Ser Ile Asn Phe Pro Ser Phe Phe Thr Thr Val Met Thr Cys Ala
            100                 105                 110
Tyr Leu Ala Gly Leu Ser Met Leu Ser Thr Val Ser Thr Glu Arg Cys
        115                 120                 125
Leu Ser Val Leu Trp Pro Ile Trp Tyr Arg Cys Arg Arg Pro Arg His
    130                 135                 140
Leu Ser Ala Val Val Cys Val Leu Leu Trp Ala Leu Ser Leu Leu Leu
145                 150                 155                 160
Ser Ile Leu Glu Gly Lys Phe Cys Gly Phe Leu Phe Ser Asp Gly Asp
                165                 170                 175
Ser Gly Trp Cys Gln Thr Phe Asp Phe Ile Thr Ala Ala Trp Leu Ile
            180                 185                 190
Phe Leu Phe Met Val Leu Cys Gly Ser Ser Leu Ala Leu Leu Val Arg
        195                 200                 205
Ile Leu Cys Gly Ser Arg Gly Leu Pro Leu Thr Arg Leu Tyr Leu Thr
    210                 215                 220
Ile Leu Leu Thr Val Leu Val Phe Leu Leu Cys Gly Leu Pro Phe Gly
225                 230                 235                 240
Ile Gln Trp Phe Leu Ile Leu Trp Ile Trp Lys Asp Ser Asp Val Leu
                245                 250                 255
Phe Cys His Ile His Pro Val Ser Val Val Leu Ser Ser Leu Asn Ser
            260                 265                 270
Ser Ala Asn Pro Ile Ile Tyr Phe Phe Val Gly Ser Phe Arg Lys Gln
        275                 280                 285
Trp Arg Leu Gln Gln Pro Ile Leu Lys Leu Ala Leu Gln Arg Ala Leu
    290                 295                 300
Gln Asp Ile Ala Glu Val Asp His Ser Glu Gly Cys Phe Arg Gln Gly

305                 310                 315                 320
Thr Pro Glu Met Ser Arg Ser Ser Leu Val
                325                 330

<210> 3
<211> 24
<212> DNA
<213> Homo sapiens

<400> 3
accatggatc caaccacccc ggcc                                              24

<210> 4
<211> 25
<212> DNA
<213> Homo sapiens

<400> 4
ctacaccaga ctgcttctcg acatc                                             25

<210> 5
<211> 23
<212> DNA
<213> Homo sapiens

<400> 5
gcaccagtgg aggttttctg agc                                               23

<210> 6
<211> 21
<212> DNA
<213> Homo sapiens

<400> 6
tcatcacagt ggtgaagaag c                                                 21

## Description

### Technical Field if the Invention

This invention relates to the identification of an orphan G-protein coupled receptor MRGX2 (and variants thereof) as a receptor of neuropeptides such as but not limited to cortistatin, somatostatin, Bam 13-22, α-MSH, neuropeptide FF, dynorphin A and substance P, and the use of such neuropeptides (and structurally related molecules) as modulators for MRGX2. It also relates to screening methods to identify agonists and antagonists for MRGX2.

### Background to the Invention

G-protein coupled receptors are a large superfamily of integral membrane proteins, involved in a broad range of signalling pathways. Most G protein-coupled receptors are characterised by 7 transmembrane-spanning helices, and are therefore also called 7-transmembrane receptors (7TMs). There are at least several hundred members of this family, which include receptors responding to a wide range of different stimuli, including peptides, biogenic amines, lipids, neurotransmitters, hormones, nucleotides, sugar-nucleotides, cytokines, etc. Structurally, the receptors of this family consist of an extracellular amino terminal domain, seven membrane-spanning hydrophobic regions, six loop regions, three of which are extracellular, the other three being intracellular, and an intracellular carboxy terminal domain. The hydrophobic regions show considerable homology between the different members of this family, whereas the loop regions, as well as the amino and carboxy terminal domains, are quite diverse, showing high homology only amongst closely related receptor subtypes.

Through analysis of sequence homology, this superfamily can be subdivided into families and subfamilies; for example, in general those receptors with peptide ligands show more sequence homology to other GPCRs which respond to peptide ligands than to those which respond to for example, bioactive amines or lipids etc.

The conserved structure and hydrophobicity profile of this receptor family has allowed the identification and cloning of many members of this family, both through cloning experiments, e.g. degenerate polymerase chain reaction or cDNA library screening with probes derived from GPCR sequences using low stringency, and through bioinformatic mining of sequence databases. For many of these receptors, it is yet unclear what their natural ligand and function are. These receptors are referred to as 'orphan receptors'. Through detailed analysis of their sequences and comparison with the sequences of GPCRs with known ligands, hypotheses can be generated as to the class of ligands that the orphan receptor may respond to.

However, these predictions are often tenuous, and it is still difficult to identify the ligand that such an orphan receptor may respond to. This is mainly because stimulation of GPCRs can lead to a multitude of different intracellular effects, which are mediated through the coupling to different G proteins. The G proteins in turn act on a whole range of signalling proteins, including phospholipase C, adenylate cyclase and ion channels, and thereby initiate a cascade of events in the cells.

G proteins are heterotrimeric complexes, containing an α-, and a βγ-subunit. At least 20 human genes are known to encode the GTP-binding α-subunits. The α-subunit family is divided into four subfamilies on the basis of their homology:
- The Gₛ family is typically able to stimulate adenylate cyclase upon agonist binding to the receptor coupling to it, and therefore lead to an increase in intracellular cyclic AMP.
- The G_{q} family, which includes G_{qα}, G_{11α}, G_{14α}, G_{16α}, lead to activation of phospholipase C.
- G₁₂ and G₁₃ appear to regulate classes of small Mr G proteins and Na⁺-H⁺ exchange.
- The Gᵢ family typically mediate inhibition of adenylate cyclase. More than half of the α-subunits are members of the Gᵢ family and include the ubiquitously expressed and nearly identical G_{i1α}, G_{i2α} and G_{i3α}, as well as several with a limited expression, such as G_{zα,} G_{oα} and transducin.

Furthermore, there are at least 5 genes encoding the β-subunits and at least 11 genes encoding y-subunits.

Briefly, initiation of signal transduction cascades involving G proteins requires the binding of an agonist ligand to a G protein-coupled receptor (GPCR). This results in the stabilisation of conformations of the GPCR that increase the rate of dissociation of the bound GDP from the nucleotide binding pocket of G_{α} subunit of a cognate G protein. Binding of GTP is thus allowed. With GTP bound, the G protein dissociates into GTP-G_{α} and G_{βγ} moieties and can hence regulate directly or otherwise the activity of several enzymes, which generate intracellular signalling molecules (e.g. cyclic AMP), or the probability of opening a range of ion channels (Gilman, A.G. (1987) Annu Rev. Biochem. 56, 614-649). Following G protein activation, hydrolysis of bound GTP by intrinsic GTPase activity terminates its ability to regulate its effectors and leads to α-GDP reassociation with βγ. The G protein therefore serves a dual role, as a relay transmitting the signal from the receptor to the effector, and as a clock that controls the duration of the signal. Therefore, depending on which G protein the GPCR couples to, very different intracellular effects can result, e.g. stimulation or inhibition of adenylate cyclase, leading to increased or decreased cyclic AMP concentrations in the cells, opening of ion channels, etc.

It is difficult to predict from the sequence of the GPCR which G protein it will couple to, and in some cases the resulting intracellular effects are difficult to measure. Furthermore, the usual host cells used for heterologous expression of cloned GPCRs only express certain G proteins; if the G protein that the orphan GPCR couples to is not expressed in these cells, the binding of an agonist to this receptor will not have a measurable effect, making it unlikely that one could find the agonist(s) for the GPCR in such a system.

It was therefore necessary to engineer specialised host cell lines, expressing various heterologous G proteins, to enable the establishment of host cell systems that can lead to measurable responses for many of the different GPCRs. There are also promiscuous G proteins, which appear to couple several classes of GPCRs to the phospholipase C pathway, such as G_{α15} or G_{α16} (Offermanns, S & Simon, M.I. (1995) J. Biol. chem. 270, 15175-15180), or chimeric G proteins designed to couple a large number of different GPCRs to the same pathway, e.g. phospholipase C (Milligan, G & Rees, S (1999) Trends in Pharmaceutical Sciences 20, 118-124). Host cell lines engineered to express one of these G proteins are more likely to give measurable responses to agonists for a variety of GPCRs than those expressing only their endogenous G_{α} subunits.

GPCRs are very important targets for pharmaceutical intervention, and many agonists and antagonists of the GPCRs of known functions are important for the treatment of diseases. Examples include but are not limited to, β₁-adrenergic receptor antagonists used for treating hypertension, angina and heart failure, dopamine D2 antagonists used for the treatment of schizophrenia, and β₂-adrenergic receptor agonists used for the treatment of asthma. Over the past 15 years, nearly 350 therapeutic agents targeting GPCRs have been introduced onto the market, and it is estimated that around 30% of clinically prescribed drugs function as either agonists or antagonists at GPCRs.

Therefore, it is likely that amongst the orphan receptors, there are further therapeutic targets which can play a key role in treating or preventing diseases or dysfunctions, including, but not limited to, sleep disorders, pain, cancers, obesity, eating disorders, hypertension, hypotension, heart failure, incontinence, asthma, chronic bronchitis, angina, ulcers, psychotic and neurological disorders, viral infections including HIV-1 or HIV-2, other infections, inflammatory conditions, sexual dysfunction, urogenital disorders and many other disorders, diseases or dysfunctions. It is therefore clearly highly desirable to find the ligand and the physiological function for each GPCR.

MRGX2 was mentioned in Dong et al (2001) Cell 106, 619-632, but its sequence was only disclosed in supplementary information available on the publisher's web site (http://www.cell.com/supplemental/106/5/619/DC 1/seq data.htm), and no characterisation of this receptor is presented in this publication. The sequence of this receptor is also disclosed in several patent applications, e.g. WO 01/19983, WO 01/48015, or WO 01/16159. However, to our knowledge, no endogenous ligand for this receptor has been disclosed to date.

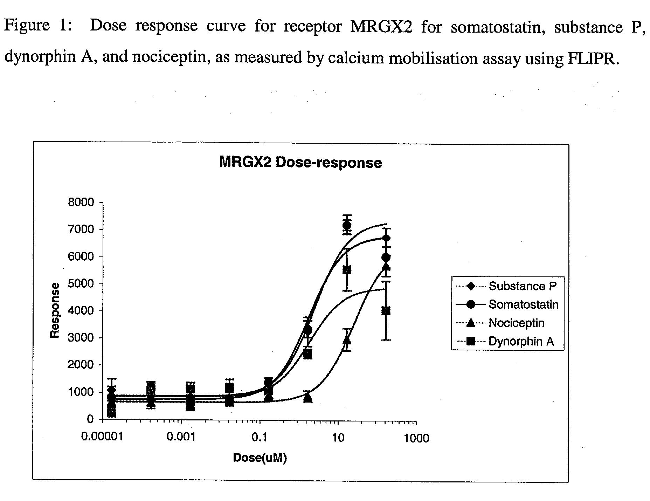

MRGX2 is one of four human genes that Dong et al identified using BLAST and hidden markov models searching the CELERA database with the murine MRGA1 sequence (GenBank accession number AY042191). The family of human receptors share high sequence homology in excess of 65% and the phylogenetic analysis clearly demonstrates that there are no clear orthologous pairs (see Fig. 1 in Dong et al (2001) Cell 106, 619-632) Within the public domain there have been a number of additional sequences deposited termed either Dorsal Root Receptors DRR1-6 or Sensory Neurone Sensing Receptors SNSR 1-6 (GenBank accession numbers AF474987, AF474988, AF474989, AF474990, AF474991, AF474992), MRGX2 being additional to these mentioned. However, from the SNSR1-6 sequences it can be established that there are only 3 rather than 6 distinct genes. This was concluded from a number of bioinformatic searches. Firstly, searches using BLAST against the CELERA database showed that SNSR1 and 2, SNSR3 and 4, and SNSR5 and 6 hit an identical loci respectively, and in addition searches of the EST databases (Incyte Genomic) identified clones from SNSR1 and 2, SNSR3 and 4, and SNSR5 and 6 with identical 3' and 5' untranslated regions respectively, this data therefore supports there being only 3 rather than 6 genes that encode the SNSR receptors. In addition a further member of the family hGPCR45 (AB083627), 41% identical has been identified which we have renamed MRGX5 to keep in line with current nomenclature.

It is interesting to speculate as to the significance of the sequence diversity within this receptor family, it may elude to the fact that the cognate endogenous ligand has yet to be identified from the human genome or that the divergent nature of activating ligands allows this family to regulated in a graded manor within functionally equivalent cell types. Strikingly all the human receptors are located on chromosome 11 which also contains 50% of all the human olfactory receptor genes (Glusman et.al. (2001) Genome Research 11, 685-702) and there may be similarities between olfactory receptor functional regulation and this family which remains to be proven.

We have identified several neuropeptides which stimulate this receptor:

The ligand with the highest affinity is Cortistatin (CST), which is a cyclic peptide derived from a prepropeptide of 114 residues in man (Lecea et.al (1997) Genomics 42, 499). It has been demonstrated that by proteolytic cleavage of the precursor at least 4 peptides can be identified, namely hCST21, hCST31, hCST29 and hCST17. Sequence analysis has shown that CST is closely related to Somatostatin (SST) at both the gene structure and the peptide level, including the motif FWKT which has been shown to be essential for SST binding to its receptor (Veber et.al. (1979) Nature 280, 512). Both CST and SST are members of a wider family of neuropeptides totaling approximately 20 that have varying degrees of sequence, structural and evolutionary relatedness; these include opioid, vasopressin, oxytocin, corticotropin-releasing factor, urotensin II, melanin concentrating hormone, neuropeptide Y and others (Conlon et.al (1997) Regul.Pept 69, 95, Darlison et.al. (1999) Trends Neurosci 22, 81). It has also been shown that SST and analogues are able to activate GPR14 albeit at lower concentration than urotensin II itself. However, it has been shown that CST does not bind GPR14 or any known opioid receptor (Connor et.al (1997) Br. J. Pharmacol 122, 1567, Nothacker et.al (1999) Nat Cell Biol 1, 383).

CST was first identified using the technique of subtractive hybrisation to identify specific rat brain genes (Lecea et.al. (1996) Nature 381, 242). The identified protein was named precortistatin in recognition of it predominant expression in the cortex. It has been shown that despite the high similarity between CST and SST they are products from distinct genes (Lecea et.al. (1997) Genomics 42, 499), suggesting these occurred due to an early gene duplication event. Interestingly the chromosome localistion of CST, 1p36, has been shown to contain the region that encodes a neuroblastoma susceptibility gene. CST has been cloned from mouse, rat and human and there is high sequence homology between species: 86% between mouse and rat and 71% between human and rat at peptide level (Lecea. et.al (1997) Genomics 42, 499). Interestingly in mouse it has been shown that only one physiologically relevant peptide is cleaved (mCST14) due to a genetic deletion that ablates a protease cleavage site.

CST is expressed in man in fetal heart, fetal lung, prostate, colon and many tumors, as shown by analysis of EST databases. In-situ evidence from mouse and rat shows expression in cerebral cortex and hippocampus with weaker signals in striatum and olfactory bulb but not in hypothalamus, midbrain, spinal cord, thalamus or cerebellum (Lecea et.al. (1996) Nature 381, 242; Lecea et.al. (1997) J. Neurosci. 17, 5868; Lecea et.al. (1997) Genomics 42, 499). Co-localisation studies have demonstrated that CST is only expressed in GABAergic interneurons similar to SST but not completely overlapping thus populations of neurons in the brain contain either or both statins (Lecea et.al. (1997) J. Neurosci. 17, 5868).

As expected the pharmacology of CST is similar to SST. CST does have high affinity for the five known SST receptors with EC50 values ranging from 0.25 - 0.9nM comparable to SST (Fuhrmann et.al (1990) Nucl. Acids Res 18, 1287; O'Dowd et.al. (1995) Genomics 28, 84). Expression analysis of the CST peptide has shown this to be regulated by the circadian cycle and sleep perturbations suggesting a physiological function of CST as a modulator of sleep. Evidence to support this demonstrates that CST is synthesized by cortical GABAergic interneurons, which increases during sleep deprivation (4 fold), selectively enhances slow wave sleep and decreases neuronal activity. Further, CST impedes the effects of acetylcholine, which is itself a REM sleep inducer (Lecea et.al (1998) Soc. NeuroSci. Abstr.24 p1429). The effects of CST on sleep cannot be wholly accounted for by the action of CST on SST receptors, suggesting that a CST specific receptor may exist. Further evidence to support this shows that ICV injection of CST results in hypomotility compared to hypermotility if SST was administered, and the effects of CST and SST on REM sleep are also reversed.

In mice, CST has also been shown to impair post-training memory processing, and appears to be connected with stroke (Flood J.F. et al (1997) Brain Res 775, 250-252). In humans, a recent report suggests that CST has some endocrine activities, it was found to interact with GHRH and ghrelin which provides a possible link to diabetes and cancer.

Our results demonstrate that CST is an endogenous ligand for MRGX2; although there is no significant sequence homology between SST receptors and MRGX2 one could speculate that the key residues that confer ligand specificity will be conserved. This is not unheard of in the field. A further example would be the low sequence homology between histamine H2 receptor and H3 receptor being ∼ 25% but they both bind histamine with high affinity.

An alternative ligand with high affinity is Somatostatin (SST) which is a cyclic peptide hormone of 14 amino acids (there is also a 28 amino acid form) having numerous endocrine functions which, like many hormones, is cleaved from a larger precursor protein. Somatostatin inhibits the pituitary secretion of growth hormone, the pancreatic secretion of glucagon and insulin, and the secretion of gastrin from the gut. Somatostatin also acts as neurotransmitter /neuromodulator (see S. J. Hocart et al. (1998) J. Med. Chem. 41, 1146-1154 for a general discussion). The biological effects of somatostatin are apparently all inhibitory in nature, and are elicited upon binding to the surface of a target cell. The known receptors for somatostatin are integral membrane proteins, and are G-protein-coupled. It is believed that upon binding of somatostatin to the receptor, the receptor undergoes a conformational change facilitating its interaction with a G-protein at the cytoplasmic face of the receptor. This facilitates binding or release of GTP/GDP at the G protein, and leads to further activation and signalling events inside the cell. In particular, somatostatin binding at its own G-protein-coupled receptor is negatively coupled to adenylyl cyclase activity, which is necessary for the production of cyclic AMP. Thus, these further signalling events directly oppose mechanisms (for example, as mediated by calcium ions or cyclic AMP) whereby GHRP (growth hormone related peptide) and GHRH (growth hormone related hormone) would otherwise trigger extracellular secretion of growth hormone from cytoplasmic storage granules. For a general review thereof, see The Encyclopedia of Molecular Biology, J. Kendrew, ed., Blackwell Science, Ltd. 1994, at page 387. The effects of somatostatin on target cells are mediated by at least 5 classes of receptors (sst1-sst5). Although the receptors may have similar affinity for somatostatin, they are differentially expressed in different tissues, and so positioned, interact, directly or indirectly, with different intracellular signalling components. This tissue specificity of receptor expression accounts in large measure for the different effects of somatostatin in different target cell types. The known somatostatin receptors are found, for example, in tissues of the anterior pituitary, other brain tissues, the pancreas, the lung, on lymphocytes, and on mucosa cells of the intestinal tract.

The MRGX2 receptor also shows a response to Dynorphin-A (Dyn A), which is an endogenous opioid receptor agonist of 13 amino acids. Dyn A was discovered in 1975 when an opioid peptide from porcine pituitary having properties quite different from those of β-endorphin was identified and isolated. This peptide was found to have a lower apparent molecular weight and higher basicity. It also exhibited a more persistent effect in the guinea pig ileum bioassay than β-endorphin. Another interesting finding was that the biological activity of this novel substance was completely resistant to cyanogen bromide treatment, indicating that no methionine residue was essential for its activity. Finally, the peptide was purified and the sequence of its 13 N-terminal residues determined by microsequencing technology. The biological activity of this purified peptide was quite interesting. In the GPI assay, it was the most potent among all the other naturally occurring endogenous opioid peptides hitherto known, being 50 times more potent than β-endorphin, 700 times more potent than [Leu]-enkephalin and about 200 times more potent than normorphine. In order to indicate its extraordinary potency, the natural peptide was named dynorphin (dyn-signifying power).

Dynorphin has been reported to be involved in analgesia; however, reports concerning the analgesic efficacy of Dyn A have been peculiar. Unlike other opioid peptides, Dyn A and related peptides when administered ICV (intracerebroventricularly) have been found to be ineffective in thermal analgesic assays using heat such as rat tail flick or hot plate tests. However, in non-thermal and mechanical analgesic assays such as flinch-jump response to foot shock, the paw pressure test or the tail pinch test, these peptides have been reported to have a moderate analgesic effect intracerebroventricularly. Reports of spinal administration have also been conflicting. In the writhing assay Dyn A(1-13) and other smaller fragments have been found to be very potent and were not affected by the presence of naloxone or nor-Binaltorphimine (norBNI). This indicates that the spinal analgesia is mediated by non-opioid receptors. The analgesia resulting from spinal or supraspinal administration of Dyn A(1-13) is accompanied by non-opioid effects (motor disturbances, spinal cord injury and hind limb paralysis) that impair the assessment of its antinociceptive activity. The motor effects of Dyn A were attributed to a possible facilitation of excitatory amino acid activity. The involvement of NMDA receptor rather than opioid receptors in the motor effects of Dyn A(1-13) was accompanied by local ischemia, a phenomenon known to induce the secretion of excitatory amino acids. Despite these conflicting findings, Dyn A(1-13) and Dyn A(1-10) amide have been used successfully for the treatment of intractable pain in cancer patients. Thus, even though Dyn A was first described as a potent endogenous opioid peptide, its physiological or pathophysiological function was proposed to also depend on its interaction with non-opioid receptors as well.

Dyn A has also been known to regulate cardiovascular functions. This can be observed at both the CNS and peripheral levels and is mediated by both opioid and non-opioid receptors. It is reported that intravenous administration of Dyn A(1-13) and Dyn A(1-10)-NH₂ in anaesthesized animals produce hypotension associated with bradycardia both responses being blocked by naloxone and MR-2266, a κ opioid receptor antagonist. However, in conscious animals, intravenous administration of Dyn A(1-13) evokes hypertension and bradycardia. Interestingly, these effects are not antagonized by MR-2266 but by the Arg⁸-vasopressin (AVP-V1) antagonist d(Ch205Tyr(Me))AVP suggesting a possible interaction of Dyn A with the AVP-V1 receptor. Taken together, these data indicate that IV administration of Dyn A(1-13) in rats evoke both depressor and pressor effects, depending on the state of consciousness of the animal and these effects are most likely mediated by opioid and non-opioid receptors, respectively. Dynorphin A related peptides have also been shown to modulate the release of norepinephine from the nerve terminals. Further, Dyn A and related peptides cause multiple effects on heart as well. Several studies have also indicated that Dyn A and κ opioid receptors are involved in hemorrhagic shock and ischemia reperfusion injury. It is interesting to note that dynorphin A and related peptides induce two opposite effects: one, hypotension resulting from presynaptic or post-synaptic opioid receptor stimulation, leading to inhibition of norepinephrine release from synaptic nerve terminals; the other being hypertension caused by a blockade of the uptake of norepinephine via the stimulation of dynorphin specific non-opioid receptor. Therefore, it can be concluded that the CNS effect of dynorphin is mediated by a dual mechanism involving opioid as well as non-opioid receptors. However, a clear-cut mechanism for the stimulation of opioid and non-opioid receptors by dynorphin is not yet fully known.

Modulation of the hosts immune system by opioids has been known as one of the most important biological activity of this class of compounds. Dyn A has also been studied to have a possible effect on the immune system on mice. It has been observed that Dyn A when given to mice showed enhancement of phagocytosis in mouse peritoneal macrophages in a dose dependent manner. The study also showed that Dyn A(1-13) and Dyn A(6-17) were able to enhance phagocytosis less effectively than Dyn A in similar experiments. It is interesting to note that naloxone did not inhibit the enhancement of phagocytosis induced by Dyn A indicating the involvement of possibly other receptors (non-opioids) for eliciting the immunomodulatory effect.

We have also found that the MRGX2 receptor shows a response to substance P. Since its discovery 80 years ago, substance P has been known to play a role in the contraction of smooth muscle, such as the muscle tissue of the bladder, digestive system, and other internal organs. In the brain, this peptide, the most abundant member of a group known as neurokinins, functions as a neuromodulator - a chemical that transmits messages between neurons and modifies the overall responsiveness of a nerve circuit. The known substance P receptor is a G-protein coupled receptor, also known as NK1 receptor.

The MRGX2 receptor also responds to neuropeptide FF (NPFF), which is an octapeptide isolated from bovine brain in 1985 by Yang et al ((1985) Proc. Natl. Acad. USA 82, 7757-7761) using antibodies to the molluscan neuropeptide FMRFamide (FMRFa). FMRFamide-like immunoreactivity was observed in rat brain, spinal cord, and pituitary, suggesting the existence of mammalian homologs of the FMRFa family of invertebrate peptides. The isolation of NPFF named for its N- and C-terminal phenylalanines (also called F8Famide) and a second mammalian peptide, NPAF (also called A18Famide), confirmed the existence of mammalian family of peptides sharing C-terminal sequence homology with FMRFa. Molecular cloning has revealed that NPFF and NPAF are encoded by the same gene and cleaved from a common precursor protein. Studies of the localization, radioligand binding, and function of NPFF-like peptides indicate they are neuromodulatory peptides whose effects are likely to be mediated by G protein-coupled receptors. NPFF, also called "morphine modulating peptide", is an endogenous modulator of opioid systems with effects on morphine analgesia, tolerance, and withdrawal. NPFF appears to represent an endogenous "anti-opioid" system in the CNS acting at specific, high- affinity receptors distinct from opiate receptors. Endogenous NPFF has been suggested to play a role in morphine tolerance: agonists of NPFF precipitate "morphine abstinence syndrome" (i.e. symptoms of morphine withdrawal) in morphine-dependent animals, while antagonists and anti-NPFF IgG restore morphine sensitivity and ameliorate symptoms of withdrawal. NPFF antagonists potentially could be useful as therapeutic agents to prevent the development of morphine tolerance, and to treat opiate addiction.

NPFF has also been suggested to participate in the regulation of pain threshold, showing both "anti-opiate" effects and analgesic effects depending on test system and route of administration. As an anti-opiate, NPFF has been shown to inhibit morphine- and stress-induced analgesia, whereas anti-NPFF IgG (which blocks the biological activity of NPFF) potentiates these two phenomena. An NPFF antagonist may be clinically useful in potentiating the analgesic effects of morphine, allowing use of lower doses without the development of tolerance. NPFF agonists may also exhibit analgesic activity in some model systems. The analgesia elicited by NPFF is typically sensitive to naloxone, indicating that it is mediated by release of endogenous opioid peptides. The interaction of NPFF and opioid systems in regulating pain pathways is thus complex and may involve multiple mechanisms and sites of action. NPFF has additional biological activities in accord with its pattern of expression in the nervous system. NPFF peptide localization in rat CNS was examined using specific antibodies. The highest levels of NPFF are found in spinal cord and posterior pituitary; pituitary NPFF is believed to originate in the hypothalamus. In the brain, immunoreactive cell bodies are found in two major cell groups: medial hypothalamus (between dorsomedial and ventromedial) and nucleus of the solitary tract. Immunoreactive fibers are observed in lateral septal nucleus, amygdala, hypothalamus, nucleus of solitary tract, ventral medulla, trigeminal complex, and dorsal horn of spinal cord. This localization pattern is consistent with a role for NPFF in sensory processing and modulation of opioid systems. In addition, its presence in the hypothalamus and other limbic structures could subserve roles in the regulation of appetitive and affective states. In the periphery, NPFF-like immunoreactivity (as well as NPFF binding) has been observed in the heart. In addition, injection of NPFF raises blood pressure in rats. These observations, combined with the colocalization of NPFF with catecholaminergic neurons in the nucleus of the solitary tract, suggest that NPFF is involved in central and peripheral cardiovascular regulation. The ability of NPFF peptides to modulate the opioid system raised the possibility that NPFF interacts directly with opiate receptors. However, radioligand binding assays using a tyrosine-substituted NPFF analog [¹²⁵I)Y8Fa demonstrate that NPFF acts through specific high affinity binding sites distinct from opiate receptors that are sensitive to inhibition by guanine nucleotides.

The MGX2 receptor shows a potent response to HS024 (Acetyl-Cys³,Nle⁴,Arg⁵,D-2-Nal⁷,Cys¹¹)- α-MSH (3-11) amide, and also responds to α-MSH (melanocyte stimulating hormone) and γ-MSH at a concentration of 1µM. α-MSH modulates many forms of inflammation by direct action on peripheral inflammatory cells, on inflammatory cells in brain such as glial cells, and on CNS receptors that activate descending anti-inflammatory neural pathways. The multiple actions of α-MSH suggests that it modulates its effects via several biochemical mechanisms including inhibition of NFκB (Lipton et al (1999) Ann NY Acad Sci 885, 173-182). α-MSH has also shown inhibitory influences against gram-positive bacteria, and reduces HIV replication in chronically and acutely infected human monocytes (Catania et al (2000) Ann NY Acad Sci 917, 227-231).

HS024 was synthesised as a novel cyclic α-MSH analogue for use as an antagonist at the melanocortin (MC) receptor family (Kask et al (1998) Endocrinology 139, 5006-5014). There are five classes of MC receptors designated MC1 - 5 receptor. HS024 has been shown to antagonise an α-MSH induced cAMP response in cells expressing MC 1,3,4 and 5 receptors but shows a 20 fold selectivity and a high affinity (Kᵢ - 0.29 nM) for the MC4 receptor. We have shown HS024 to act as an agonist at MRGX2. I.c.v. administration of HS024 in free feeding rats caused a dose dependant increase in food intake with a maximal response at 1nmol dose. Some rats treated at this dose developed opthalamus and barrel rolling behaviour. Failure of higher doses of cylic α-MSH analogues to modulate feeding is suggested to be due to sedative effects. It is interesting to speculate that this effect could be due to interaction with MRGX2, especially when considered in conjunction with the evidence of the sleep inducing effects of cortistatin via a non-somatostatin receptor mediated mechanism.

Bovine adrenal medulla peptide 22 (BAM 22), and fragments thereof, act as agonists at MRGX2. BAM22 is a 22 amino acid peptide that is produced by proteolytic cleavage of a proenkephalin A precursor, and has been identified as the endogenous ligand for the SNSR3 & 4 (MRGX1) receptors with EC₅₀'s of 13 and 16nM respectively (Lembo et al, (2002) Nature Neuroscience 5, 201-209). BAM22 is also known to bind to all three opioid receptor subtypes with high affinity and can compete with and mimic the effects of the opioid drugs. BAM22 is distributed throughout the brain, gastrointestinal tissue, pancreatic islets of Langerhans and plasma. BAM 8-22 has been shown to modulate nociceptive responses. Rats treated with BAM 8-22 exhibited enhanced touch response flexor reflex, and induction of mechanical and thermal hypersensitivity (Cao et al,10th World Congress on Pain, August 17-22, 2002). As well as functions in nociception, BAM22 has also been implicated in inhibition of reflex urinary bladder contractions, inhibition of gastrointestinal transit (Porreca et al (1985) Adv. Pain Res. Ther.) and development of spontaneous hypertension (Hoegler et al, Am. J. Hypertension (1989) 2(7) 542-548).

The discovery that MRGX2 is activated by cortistatin, somatostatin, Bam 13-22, αMSH, neuropeptide FF, dynorphin A and substance P, and the expression of this receptor in dorsal root ganglion, vascular smooth muscle, brain, heart, and inflammatory cell types suggests that MRGX2 may be the receptor responsible for the mechanism by which these ligands exert their physiological function.

Given the biological effects of the ligands identified, in connection with the tissue distribution of this receptor, agonists and antagonists for this receptor could be therapeutic agents for the treatment of all types of sleep disorders, pain, stroke, memory disorders, diabetes, cancer, obesity, cardiovascular disorders, depression, sexual dysfunction, urinary tract and bladder disorders, anti-infective treatments, gastrointestinal disorders, and other disorders.

### Aspects of the Invention

One aspect of the invention is the use of neuropeptides, preferably of cortistatin, somatostatin, Bam 13-22, α-MSH, neuropeptide FF, dynorphin A and substance P, or analogues or mimetics of any one of these neuropeptides as a ligand for receptor MRGX2, preferably as a modulator of receptor MRGX2, even more preferably as an agonist of receptor MRGX2. Preferably, the neuropeptides that act as ligands, preferably as modulators, even more preferably as agonists, of receptor MRGX2 are neuropeptides that form internal disulfide linkages and are therefore cyclic neuropeptides. In a preferred aspect of the invention, neuropeptides, preferably cortistatin, somatostatin, Bam 13-22, α-MSH, neuropeptide FF, dynorphin A or substance P or analogues or mimetics of any one of these neuropeptides are used to elicit a functional response on receptor MRGX2. A preferred aspect is the use of cortistatin, somatostatin, Bam 13-22, α-MSH or neuropeptide FF as a ligand for MRGX2, more preferably as an agonist for MRGX2.

A further aspect of the invention is a method for identifying a compound that modulates a novel neuropeptide-mediated process, comprising a) contacting a sample of MRGX2 with a neuropeptide, preferably a cyclic neuropeptide, such as cortistatin, somatostatin, Bam 13-22, α-MSH, or neuropeptide FF in the presence or absence of a test compound or test compounds, b) determining the biological effects thereof. In a typical assay, the test compound will bind, and have effects, at the same site on MRGX2 as cortistatin or one of the other neuropeptides normally bind, although the skilled practitioner will recognise that this need not always be so. The methodologies of the present invention may also be used to identify compounds acting at sites on MRGX2 remote to the cortistatin or neuropeptide binding site. In one aspect, the method comprises (a) contacting MRGX2 with ligand (cortistatin, somatostatin, Bam 13-22, α-MSH, neuropeptide FF, dynorphin A or substance P or an analogue or mimetic of any one of these neuropeptides) in the presence of a test compound; and (b) measuring the amount of ligand bound to MRGX2 in the absence of a test compound, such that if the amount of bound ligand measured in (a) and (b) is different, then a compound that modulates the binding of ligand to MRGX2 is identified. In one embodiment, the MRGX2 contacted in step (a) is on a cell surface. In another embodiment, the MRGX2 is immobilised on a solid surface. In another embodiment, the solid surface is a microtiter dish. In yet another embodiment, the MRGX2 is a membrane preparation used in suspension.

Therefore, one aspect of the invention is a method of screening for compounds that are ligands, preferably modulators, even more preferably agonists or antagonists, of MRGX2, using cortistatin, somatostatin, Bam 13-22, α-MSH, neuropeptide FF, dynorphin A or substance P or an analogue or mimetic of any one of these neuropeptides as ligand. Other neuropeptides, preferably other cyclic neuropeptides, or analogues or mimetics thereof, may also be found to be suitable ligands for use in methods of screening for compounds that are ligands for MRGX2. This extends also to all further aspects of the invention mentioned below involving a method of screening for compounds that bind to MRGX2.

A further aspect of the invention is a method of screening for compounds that are ligands, preferably modulators, more preferably agonists or antagonists of novel neuropeptide receptors, including but not limited to the following steps:
(a) contacting a sample of MRGX2 with a ligand selected from cortistatin, somatostatin, Bam 13-22, α-MSH, neuropeptide FF, dynorphin A or substance P or an analogue or mimetic of any one of these neuropeptides;
(b) contacting a similar sample of MRGX2 with the same ligand as in step (a) and a test compound or mixture of test compounds; and
(c) comparing the results in (a) and (b) to determine whether the binding of the ligand is affected by the presence of the test compound or mixture of test compounds.

Preferably the method includes but is not limited to the following steps:
(a) contacting a sample of MRGX2 with a ligand, selected from cortistatin, somatostatin, Bam 13-22, α-MSH, neuropeptide FF, dynorphin A or substance P or an analogue or mimetic of any one of these neuropeptides, with a detectable label attached, in the presence or absence of a test compound or mixture of test compounds;
(b) measuring the amount of label bound, whereby a reduction of bound label in presence as compared to the amount of bound label in the absence of a test compound or mixture of test compounds indicates that the test compound or one or more test compounds present in the mixture of test compounds bind(s) to novel neuropeptide receptor.

Another aspect of the invention is the use of cortistatin, somatostatin, Bam 13-22, α-MSH, neuropeptide FF, dynorphin A or substance P or an analogue or mimetic of any one of these neuropeptides to modulate, preferably agonise or antagonise, the receptor encoded by a nucleotide sequence comprising SEQ ID NO 1, or variants or homologues thereof.

A further aspect of the invention is the use of a novel neuropeptide receptor, preferably a cortistatin receptor, a somatostatin receptor, a Bam 13-22 receptor, a α-MSH receptor, or a neuropeptide FF receptor, in an assay wherein said receptor comprises the protein sequence of SEQ ID NO 2, or is a variant or homologue thereof.

Another aspect of the invention is a method of expressing a novel neuropeptide receptor, preferably a cortistatin receptor, a somatostatin receptor, a Bam 13-22 receptor, a αMSH receptor, a neuropeptide FF receptor, a dynorphin A receptor or a substance P receptor, comprising transferring a suitable expression vector comprising SEQ ID NO 1 or variants or homologues thereof, into suitable host cells, and culturing said host cells under conditions suitable for the expression of the receptor. Suitable host cells are bacteria such as *Escherichia coli,* yeasts such as *Saccharomyces cerevisiae* or *Pichia pastoris,* or preferably insect cells, or more preferably, mammalian cells, most preferably the host cells are human cells. A further aspect of the invention is a method of expressing the neuropeptide receptor, comprising upregulating the expression of MRGX2 in a suitable cell. This can be achieved, for example, by insertion of a strong promoter in front of the MRGX2 gene, for example similar to the method used for the expression of erythropoietin in patent EP 411678, but other ways of upregulating MRGX2 expression are envisaged. The sample of MRGX2 in the above methods can therefore be derived from cells expressing MRGX2 naturally, cells where the expression of MRGX2 is upregulated by various means available in the art or developed in future, or from cells which have been transfected with an expression construct which contains a sequence comprising SEQ ID NO 1 or a variant or homologue thereof, or a sequence which encodes the protein sequence of SEQ ID NO 2.

A sample of MRGX2 comprises cells prepared as described above, or cells naturally expressing MRGX2, or membrane preparations prepared from any cells expressing MRGX2, or MRGX2 protein enriched or purified from such cells or membranes. The skilled person will be well aware of methods that can be used to enrich or purify MRGX2, which include affinity chromatography, size exclusion chromatography, ion exchange chromatography and other methods suitable for the separation of protein from complex mixtures.

Yet a further aspect of the invention is a method for screening for agonists of a novel neuropeptide receptor, including, but not limited to, the steps:
(a) adding a test compound or a mixture of test compounds to suitable cells expressing MRGX2,
(b) measuring whether a functional response is seen.

In a preferred aspect of the invention, the functional response is a transient rise in intracellular calcium concentration, measured by using fluorescent dyes such as Fluo-3 or Indo-1 or by other means known to the person skilled in the art; in another preferred aspect of the invention, the functional response is an increase in the rate of respiration of the cells as measured by an increase in the rate of acidification of the medium surrounding the cells as measured by microphysiometry. In yet another preferred aspect of the invention, the functional response is an increase or decrease in the cyclic AMP concentration in the cells, as measured e.g. by increased activity of a reporter gene product wherein the coding region of the reporter gene is functionally linked to a promoter comprising at least one cyclic AMP response element. Other methods to measure changes in cyclic AMP concentration in cells are well known to the person skilled in the art. In a further preferred aspect of the invention, cells are transfected with a plasmid or plasmids leading to co-expression of MRGX2 and GFP-β-arrestin complex, and an agonist of the receptor is identified by observing clustering of fluorescence, i.e. GFP-β- arrestin complex, on the cell surface. Suitable cells expressing MRGX2 are selected from cells naturally expressing MRGX2, cells where the expression of MRGX2 has been upregulated as described above, or cells where MRGX2 has been expressed by transfection with an expression construct which contains a sequence comprising SEQ ID NO 1 or a variant or homologue thereof, or a sequence which encodes the protein sequence of SEQ ID NO 2.

The invention also includes a method of screening for antagonists of a novel neuropeptide receptor, including, but not limited to, the following steps:
(a) adding a test compound or a mixture of test compounds to suitable cells expressing MRGX2,
(b) adding cortistatin, somatostatin, Bam 13-22, αMSH, neuropeptide FF, dynorphin A or substance P, or an analogue or mimetic thereof, or an agonist as identified by the methods described above;
(c) measuring whether a functional response is seen, identifying antagonists as the test compounds which reduce the functional response to the agonist.

Suitable cells expressing MRGX2 are selected from cells naturally expressing MRGX2, cells where the expression of MRGX2 has been upregulated as described above, or cells where MRGX2 has been expressed by transfection with an expression construct which contains a sequence comprising SEQ ID NO 1 or a variant or homologue thereof, or a sequence which encodes the protein sequence of SEQ ID NO 2. Examples of functional responses that can be used in this method are as described above.

Another aspect of the invention are the compounds identified by any of the methods of the invention, and pharmaceutical compositions comprising a compound identified by any of the methods of the invention.

A further aspect of the invention is a method of preparing a pharmaceutical composition which comprises determining whether a compound is a novel neuropeptide receptor agonist or antagonist using any one of the methods of the invention, and admixing said compound with a pharmaceutically acceptable carrier.

The invention is therefore, inter alia, directed to methods for screening for compounds that are useful in the treatment of neuropeptide-related disorders, preferably disorders related to this neuropeptide receptor previously called MRGX2, such as all types of sleep disorders, pain, stroke, memory disorders, diabetes, cancer, obesity, cardiovascular disorders, depression, sexual dysfunction, urinary tract and bladder disorders, anti-infective treatments, gastrointestinal disorders, and other disorders.

The invention also provides assays to further characterise the biological activity of such compounds (for example agonists or antagonists), and their resultant utilities. Certain identified compounds will be useful in the treatment and prevention of disorders and conditions in which neuropeptides such as the neuropeptides identified to activate MRGX2 participate, such as, for example, all types of sleep disorders, pain, stroke, memory disorders, diabetes, cancer, obesity, cardiovascular disorders, depression, sexual dysfunction, urinary tract and bladder disorders, anti-infective treatments, gastrointestinal disorders, and other disorders.

Another aspect of the invention is a method for detecting novel neuropeptide receptor-related disorders in a mammal comprising measuring the level of MRGX2 gene expression or of MRGX2 protein, in a patient sample, such that if the measured level differs from the level found in clinically normal individuals, then a novel neuropeptide receptor/MRGX2 related disorder is detected. Also provided are kits for measuring MRGX2, e.g. comprising an antibody specifically recognising MRGX2 (see below), or comprising the means to measure MRGX2 mRNA, such as a probe hybridising to the MRGX2 mRNA, or primers suitable for a PCR-based detection of the MRGX2 mRNA or cDNA.

By way of example, levels of MRGX2 can be detected by immunoassay like enzyme-linked immunosorbant assays (ELISAs), radioimmunoassays (RIA), immunocytochemistry, immunohistochemistry, Western blots, or other methods familiar to the skilled person. Levels of MRGX2 RNA can be detected by hybridization assays (e.g. Northern blots, *in situ*-hybridization), and MRGX2 activity can be assayed by measuring binding activities as described above *in vivo* or *in vitro.* Translocations, deletions, and point mutations in MRGX2 nucleic acids can be detected by Southern blotting, FISH, RFLP analysis, SSCP, PCR using primers that preferably generate a fragment spanning at least most of the MRGX2 gene, sequencing of MRGX2 genomic DNA or cDNA obtained from the patient, etc.

Compounds that affect MRGX2 gene activity, e.g. by affecting transcription, interfering with splicing events, or translation, can also be identified using the methods of the invention. It should also be noted that compounds that modulate signal transduction resulting from agonist binding to MRGX2 can be detected by methods of the invention, and are within the scope of the invention.

For the sake of clarity, the term "novel neuropeptide receptor" refers to the receptor for neuropeptides such as cortistatin, somatostatin, Bam 13-22, aMSH, neuropeptide FF, dynorphin A, substance P. Without wishing to be bound by theory, the neuropeptides identified to activate this receptor are all cyclic neuropeptides, i.e. they form an internal disulfide bridge.

In this document the terms "homologue" or "variant" refer to an entity having a certain homology with the amino acid sequence and nucleotide sequence specified in SEQ ID NOs 1 and 2. A homologous sequence is taken to include an amino acid sequence which may be at least 75, 85, or 90% identical, preferably at least 95 or 98% identical to the specified sequence, or a nucleotide sequence which may be at least 75, 85 or 90% identical, more preferably at least 95 or 98% identical to the specified sequence. Such sequence homology/identity can be easily assessed by publicly or commercially available bioinformatics software, such as Blast2 (Altschul, S.F. et al (1997) Nucl. Acids Res. 25, 3389-3402), or programs included in the GCG software package (Devereux et al (1984) Nucl. Acids Res. 12, 387; Wisconsin Package Version 10, Genetics Computer Group (GCG, Madison, Wisconsin), such as Bestfit or Gap.

In this document, the term "analogue" relates to any substance which is similar in structure to a reference agent, e.g. cortistatin, somatostatin, Bam 13-22, αMSH, neuropeptide FF, dynorphin A or substance P, and has a generally similar biological effect, at least under one condition of assay.

As used herein, the term "mimetic" relates to any substance (which includes, but is not limited to, a peptide, polypeptide, antibody or other organic chemical) which has the same qualitative activity or effect as a reference agent, e.g. cortistatin, somatostatin, Bam 13-22, αMSH, neuropeptide FF, dynorphin A or substance P.

The term "modulator of a receptor/enzyme" refers to any substance which binds to, and has an effect on, the respective receptor/enzyme.

The term "agonist of a receptor" refers to a substance which can stimulate the respective receptor.

The term "antagonist of a receptor" refers to a substance which can inhibit the stimulation of the respective receptor by an agonist.

The term "functional response" refers to the reaction that, for example, stimulation of a receptor leads to in a cell. In the case of G-protein coupled receptors, this can include, for example, a change in the concentration of cyclic AMP, a transient rise in intracellular calcium concentration, or an opening of an ion channel.

The term "reporter gene" refers to a gene encoding a protein that can be conveniently measured. Preferably a gene used as reporter gene is not naturally expressed in the cells used. Examples include β-galactosidase, luciferase, chloramphenicol transferase (CAT) or β-lactamase. Typically the reporter gene is inserted behind a promoter or a response element or several response elements linked to a basic promoter, and upon a stimulatory signal, the reporter gene is translated, and the activity of the corresponding enzyme is increased, which can be easily measured.

The term "compound" refers to any chemical entity, including but not limited to a small organic molecule, a peptide, a protein, a modified protein such as a glycoprotein or a lipoprotein, antibodies or fragments thereof, a nucleic acid such as DNA or RNA or modified nucleic acids, such as oligonucleotides with a modified backbone.

### Further Details about the Invention

The methods of the invention may be used to identify compounds, such as small organic molecules or peptides or proteins, for example, that modulate the interaction of the neuropeptide such as cortistatin, somatostatin, Bam 13-22, α-MSH, neuropeptide FF, dynorphin A, or substance P, or analogues or mimetics of any one of these neuropeptides. The substances which may be screened in accordance with the invention therefore also include antibodies and fragments thereof. Peptidomimetic organic compounds that bind, for example, to the extra-cellular domain (ECD) of MRGX2 and either inhibit the activity triggered by the natural ligand *(i.e.,* antagonists) or mimic the activity triggered by the natural ligand *(i.e.,* agonists), may also be screened. Additionally, organic compounds, peptides, antibodies or fragments thereof, to which the ECD (or a portion thereof) of MRGX2 is covalently attached may also bind to and therefore "neutralize" the MRGX2 ligand. Screening of such complex reagents is also within the practice of the invention.

Compounds that may be used for screening include, but are not limited to, peptides such as, for example, soluble peptides, including but not limited to members of random peptide libraries; (see, e.g., Lam et al. (1991) Nature 354, 82-84; Houghten et al. (1991) Nature 354, 84-86), and combinatorial chemistry-derived molecular library made of D- and/or L-configuration amino acids, phosphopeptides (including, but not limited to, members of random or partially degenerate, directed phosphopeptide libraries; see, e.g., Songyang et al. (1993) Cell 72, 767-778), antibodies (including, but not limited to, polyclonal, monoclonal, humanized, anti-idiotypic, chimeric or single chain antibodies, and Fab, F(ab')₂ and Fab expression library fragments, and epitope-binding fragments thereof), and small organic or inorganic molecules.

In one embodiment of the present invention, peptide libraries may be used as a source of test compounds that can be used to screen in the methods of the invention. Diversity libraries, such as random or combinatorial peptide or nonpeptide libraries can be screened for molecules that specifically bind to the MRGX2 receptor. Many libraries are known in the art that can be used, e.g., chemically synthesized libraries, recombinant (e.g. phage display libraries), and *in vitro* translation-based libraries.

Examples of chemically synthesized libraries are described in Fodor et al. (1991) Science 251, 767-773; Houghten et al. (1991), Nature 354, 84-86; Lam et al. (1991), Nature 354, 82-84; Medynski (1994) Bio/Technology 12, 709-710; Gallop et al. (1994), J. Medicinal Chemistry 37, 1233-1251; Ohlmeyer et al. (1993), Proc. Natl. Acad. Sci. USA 90, 10922-10926; Erb et al. (1994), Proc. Natl. Acad. Sci. USA 91, 11422-11426; Houghten et al. (1992) Biotechniques 13, 412; Jayawickreme et al. (1994), Proc. Natl. Acad. Sci. USA 91, 1614-1618; Salmon et al. (1993) Proc. Natl. Acad. Sci. USA 90, 11708-11712; PCT Publication No. WO 93/20242; and Brenner and Lerner (1992) Proc. Natl. Acad. Sci. USA 89, 5381-5383.

Examples of phage display libraries are described in Scott & Smith (1990) Science 249:386-390; Devlin et al. (1990) Science 249, 404-406; Christian, et al. (1992), J. Mol. Biol. 227, 711-718; Lenstra (1992) J. Immunol. Meth. 152, 149-157; Kay et al. (1993) Gene 128, 59-65; and PCT Publication No. WO 94/18318 dated August 18, 1994.

By way of examples of nonpeptide libraries, a benzodiazepine library (see e.g. Bunin et al. (1994), Proc. Natl. Acad. Sci. USA 91, 4708-4712) can be adapted for use. Peptoid libraries (Simon et al. (1992) Proc. Natl. Acad. Sci. USA 89, 9367-9371) can also be used. Another example of a library that can be used, in which the amide functionalities in peptides have been permethylated to generate a chemically transformed combinatorial library, is described by Ostresh et al. (1994) Proc. Natl. Acad. Sci. USA 91, 11138-11142).

Screening the libraries can be accomplished by any of a variety of commonly known methods. See, e.g., the following references, which disclose screening of peptide libraries: Parmley & Smith (1989) Adv. Exp. Med. Biol. 251, 215-218; Scott & Smith (1990) Science 249, 386-390; Fowlkes et al. (1992) BioTechniques 13, 422-427; Oldenburg et al. (1992) Proc. Natl. Acad. Sci. USA 89, 5393-5397; Yu et al. (1994) Cell 76, 933-945; Staudt et al. (1988) Science 241, 577-580; Bock et al. (1992) Nature 355, 564-566; Tuerk et al. (1992) Proc. Natl. Acad. Sci. USA 89, 6988-6992; Ellington et al. (1992) Nature 355, 850-852; U.S. Patent No. 5,096,815, U.S. Patent No. 5,223,409, and U.S. Patent No. 5,198,346, all to Ladner et al.; Rebar & Pabo (1993) Science 263, 671-673; and PCT Publication No. WO 94/18318.

Compounds that can be tested and identified methods described herein can include, but are not limited to, compounds obtained from any commercial source, including Aldrich (1001 West St. Paul Ave., Milwaukee, WI 53233), Sigma Chemical (P.O. Box 14508, St.

Louis, MO 63178), Fluka Chemie AG (Industriestrasse 25, CH-9471 Buchs, Switzerland (Fluka Chemical Corp. 980 South 2nd Street, Ronkonkoma, NY 11779)), Eastman Chemical Company, Fine Chemicals (P.O Box 431, Kingsport, TN 37662), Boehringer Mannheim GmbH (Sandhofer Strasse 116, D-68298 Mannheim), Takasago (4 Volvo Drive, Rockleigh, NJ 07647), SST Corporation (635 Brighton Road, Clifton, NJ 07012), Ferro (111 West Irene Road, Zachary, LA 70791), Riedel-deHaen Aktiengesellschaft (P.O. Box D-30918, Seelze, Germany), PPG Industries Inc., Fine Chemicals (One PPG Place, 34th Floor, Pittsburgh, PA 15272). Further any kind of natural products may be screened using the methods of the invention, including microbial, fungal, plant or animal extracts.

Furthermore, diversity libraries of test compounds, including small molecule test compounds, may be utilized. For example, libraries may be commercially obtained from Specs and BioSpecs B.V. (Rijswijk, The Netherlands), Chembridge Corporation (San Diego, CA), Contract Service Company (Dolgoprudny, Moscow Region, Russia), Comgenex USA Inc. (Princeton, NJ), Maybridge Chemicals Ltd. (Cornwall PL34 OHW, United Kingdom), and Asinex (Moscow, Russia).

Still further, combinatorial library methods known in the art, can be utilized, including, but not limited to: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam (1997) Anticancer Drug Des.12, 145). Combinatorial libraries of test compounds, including small molecule test compounds, can be utilized, and may, for example, be generated as disclosed in Eichler & Houghten (1995) Mol. Med. Today 1, 174-180; Dolle (1997) Mol. Divers. 2, 223-236; and Lam (1997) Anticancer Drug Des. 12, 145-167.

Examples of methods for the synthesis of molecular libraries can be found in the art, for example in: DeWitt et al. (1993) Proc. Natl. Acad. Sci. USA 90, 6909; Erb et al. (1994) Proc. Natl. Acad. Sci. USA 91, 11422; Zuckermann et al. (1994) J. Med. Chem. 37, 2678; Cho et al. (1993) Science 261, 1303; Carrell et al. (1994) Angew. Chem. Int. Ed. Engl. 33, 2059; Carell et al. (1994) Angew. Chem. Int. Ed. Engl. 33, 2061; and Gallop et al. (1994) J. Med. Chem. 37, 1233.

Libraries of compounds may be presented in solution (e.g. Houghten (1992) Bio/Techniques 13, 412-421), or on beads (Lam (1991) Nature 354:82-84), chips (Fodor (1993) Nature 364, 555-556), bacteria (U.S. Patent No. 5,223,409), spores (Patent Nos. US 5,571,698; US 5,403,484; and US 5,223,409), plasmids (Cull et al. (1992) Proc. Natl. Acad. Sci. USA 89, 1865-1869) or phage (Scott and Smith (1990) Science 249, 386-390; Devlin (1990) Science 249, 404-406; Cwirla et al. (1990) Proc. Natl. Acad. Sci. USA 87, 6378-6382; and Felici (1991) J. Mol. Biol. 222, 301-310).

Screening the libraries can be accomplished by any of a variety of commonly known methods. See for example the following references, which disclose screening of peptide libraries: Parmley & Smith (1989) Adv. Exp. Med. Biol. 251, 215-218; Scott & Smith (1990) Science 249, 386-390; Fowlkes et al. (1992) BioTechniques 13, 422-427; Oldenburg et al. (1992) Proc. Natl. Acad. Sci. USA 89, 5393-5397; Yu et al. (1994) Cell 76, 933-945; Staudt et al. (1988) Science 241, 577-580; Bock et al. (1992) Nature 355, 564-566; Tuerk et al. (1992) Proc. Natl. Acad. Sci. USA 89, 6988-6992; Ellington et al. (1992) Nature 355, 850-852; U.S. Patent No. 5,096,815, U.S. Patent No. 5,223,409, and U.S. Patent No. 5,198,346, all to Ladner et al., Rebar & Pabo (1993) Science 263, 671-673; and PCT Publication No. WO 94/18318.

Computer modeling and searching technologies permit identification of compounds, or the improvement of already identified compounds, that can modulate the interaction of, for example, cortistatin, somatostatin, Bam 13-22, α-MSH, neuropeptide FF, dynorphin A or substance P with MRGX2. Having identified such a compound or composition, the active sites or regions are identified. Such active sites might typically be ligand binding sites. The active site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.

Next, the three dimensional geometric structure of the active site (typcially a binding site) is determined. This can be done by known methods, including X-ray crystallography, which can determine a complete molecular structure. On the other hand, solid or liquid phase NMR can be used to determine certain intra-molecular distances. Any other experimental method of structure determination can be used to obtain partial or complete geometric structures. The geometric structures may be measured with a complexed ligand, natural or artificial, which may increase the accuracy of the active site structure determined.

If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.

Finally, having determined the structure of the active (binding) site, either experimentally, by modeling, or by a combination, candidate modulating compounds can be identified by searching databases containing compounds along with information on their molecular structure. Such a search seeks compounds having structures that match the determined active (binding) site structure and that interact with the groups defining the active site. Such a search can be manual, but is preferably computer assisted. These compounds found from this search are potential novel neuropeptide receptor-modulating compounds.

Alternatively, these methods can be used to identify improved modulating compounds from an already known modulating compound or ligand. The composition of the known compound can be modified and the structural effects of modification can be determined using the experimental and computer modeling methods described above applied to the new composition. The altered structure is then compared to the active (binding) site structure of the compound to determine if an improved fit or interaction results. In this manner systematic variations in composition, such as by varying side groups, can be quickly evaluated to obtain modified modulating compounds or ligands of improved specificity or activity.

Further experimental and computer modeling methods useful to identify modulating compounds based upon identification of the active (binding) sites of either MRGX2 or cortistatin, somatostatin, Bam 13-22, α-MSH, neuropeptide FF, dynorphin A, substance P, and analogs and mimetics of any one of these neuropeptides, will be apparent to those of skill in the art.

Examples of molecular modeling systems are the CHARMm and QUANTA programs (Polygen Corporation, Waltham, MA). CHARMm performs the energy minimization and molecular dynamics functions. QUANTA performs the construction, graphic modelling and analysis of molecular structure. QUANTA allows interactive construction, modification, visualization, and analysis of the behavior of molecules with each other.

A number of articles review computer modeling of drugs interactive with specific proteins, such as Rotivinen et al. (1988) Acta Pharmaceutical Fennica 97, 159-166; Ripka (1988) New Scientist 54-57; McKinaly and Rossmann (1989) Annu. Rev. Pharmacol. Toxiciol. 29, 111-122; Perry and Davies, OSAR: Quantitative Structure-Activity Relationships in Drug Design pp. 189-193 Alan R. Liss, Inc. (1989); Lewis and Dean (1989) Proc. R. Soc. Lond. 236, 125-140 and 141-162); and, with respect to a model receptor for nucleic acid components, Askew et al. (1989) J. Am. Chem. Soc. 111, 1082-1090. Other computer programs that screen and graphically depict chemicals are available from companies such as BioDesign, Inc. (Pasadena, CA.), Allelix, Inc. (Mississauga, Ontario, Canada), and Hypercube, Inc. (Cambridge, Ontario). Although these are primarily designed for application to drugs specific to particular proteins, they can be adapted to design of drugs specific to regions of DNA or RNA, once that region is identified.

MRGX2 protein, polypeptides and peptide fragments, mutated, truncated or deleted forms of the MRGX2 and/or MRGX2 fusion proteins can be prepared for a variety of uses, including but not limited to the generation of antibodies, as reagents in diagnostic assays, the identification of other cellular gene products involved in the regulation of neuropeptide-related disorders, as reagents in assays for screening for compounds that can be used as pharmaceutical reagents in the treatment of neuropeptide-related disorders. Additionally, based upon information gained from interacting MRGX2 and cortistatin, somatostatin, Bam 13-22, a-MSH, neuropeptide FF, dynorphin A, or substance P or any other neuropeptides it may bind, peptide fragments of MRGX2 can be derived which inhibit the normal binding of circulating neuropeptides to MRGX2 receptors molecules, for *in vivo* therapeutic use.

MRGX2 peptides, polypeptides, and fusion proteins can be prepared by recombinant DNA techniques. For example, nucleotide sequences encoding one or more of the four extracellular domains of the MRGX2 receptor can be synthesized or cloned and ligated together to encode a soluble extracellular domain of MRGX2. The DNA sequence encoding one or more of the four extracellular loops can be ligated together directly or via a linker oligonucleotide that encodes a peptide spacer. Such linkers may encode flexible, glycine-rich amino acid sequences thereby allowing the domains that are strung together to assume a conformation that can bind MRGX2 ligands. Alternatively, nucleotide sequences encoding individual domains within the extracellular loops can be used to express MRGX2 peptides.

A variety of host-expression vector systems may be utilized to express nucleotide sequences encoding the appropriate regions of MRGX2 to produce such polypeptides. Where the resulting peptide or polypeptide is a soluble derivative (e.g. peptides corresponding to the extracellular domain; truncated or deleted in which the transmembrane helices and/or cellular domains are deleted) the peptide or polypeptide can be recovered from the culture media. Where the polypeptide or protein is not secreted, the MRGX2 product can be recovered from the host cell itself.

The host-expression vector systems also encompass engineered host cells that express MRGX2 or functional equivalents *in situ,* i.e. anchored in the cell membrane.

Purification or enrichment of MRGX2 from such expression systems can be accomplished using appropriate detergents and lipid micelles and methods well known to those skilled in the art. However, such engineered host cells themselves may be used in situations where it is important not only to retain the structural and functional characteristics of MRGX2, but to assess biological activity, e.g. in drug screening assays.

The host-expression vector systems that may be used for purposes of the invention include but are not limited to microorganisms such as bacteria (e.g., *E. coli, B. subtilis)* transformed with recombinant bacteriophage DNA, plasmid DNA or cosmid DNA expression vectors containing MRGX2 nucleotide sequences; yeast (e.g. *Saccharomyces, Pichia)* transformed with recombinant yeast expression vectors containing MRGX2 nucleotide sequences; insect cell systems infected with recombinant virus expression vectors (e.g. baculovirus) containing MRGX2 sequences; plant cell systems infected with recombinant virus expression vectors (e.g. cauliflower mosaic virus, CaMV; tobacco mosaic virus, TMV) or transformed with recombinant plasmid expression vectors (e.g. Ti plasmid) containing MRGX2 nucleotide sequences; or mammalian cell systems (e.g. COS, CHO, BHK, 293, 3T3) harboring recombinant expression constructs containing promoters derived from the genome of mammalian cells (e.g. metallothionein promoter) or from mammalian viruses (e.g. the adenovirus late promoter; the vaccinia virus 7.5K promoter).

In bacterial systems, a number of expression vectors may be advantageously selected depending upon the use intended for the MRGX2 gene product being expressed. For example, when a large quantity of such a protein is to be produced, for the generation of pharmaceutical compositions of MRGX2 protein or for raising antibodies to the MRGX2 protein, for example, vectors which direct the expression of high levels of fusion protein products that are readily purified may be desirable. Such vectors include, but are not limited, to the *E*. *coli* expression vector pUR278 (Ruther et al. (1983) EMBO J. 2, 1791), in which the MRGX2 coding sequence may be ligated individually into the vector in frame with the lacZ coding region so that a fusion protein is produced; pIN vectors (Inouye & Inouye (1985) Nucleic Acids Res. 13, 3101-3109; Van Heeke & Schuster (1989) J. Biol. Chem. 264, 5503-5509), and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht et al. (1991) Proc. Natl. Acad. Sci. USA 88, 8972-8976). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺•nitriloacetic acid-agarose columns and histidine-tagged proteins are selectively eluted with imidazole-containing buffers.

In an insect system, *Autographa californica* nuclear polyhedrosis virus (AcNPV) is used as a vector to express foreign genes. The virus grows in *Spodoptera frugiperda* cells. The MRGX2 coding sequence may be cloned individually into non-essential regions (for example the polyhedrin gene) of the virus and placed under control of an AcNPV promoter (for example the polyhedrin promoter). Successful insertion of a MRGX2 gene coding sequence will result in inactivation of the polyhedrin gene and production of non-occluded recombinant virus (i.e. virus lacking the proteinaceous coat coded for by the polyhedrin gene). The recombinant viruses are then used to infect cells in which the inserted gene is expressed (e.g. see Smith et al. (1983) J. Virol. 46, 584; Smith, U.S. Patent No. 4,215,051).

In mammalian host cells, a number of viral-based expression systems may be utilized. In cases where an adenovirus is used as an expression vector, the MRGX2 nucleotide sequence of interest may be ligated to an adenovirus transcription/translation control complex, e.g. the late promoter and tripartite leader sequence. This chimeric gene may then be inserted in the adenovirus genome by *in vitro* or *in vivo* recombination. Insertion in a non-essential region of the viral genome (e.g. region E1 or E3) will result in a recombinant virus that is viable and capable of expressing the MRGX2 gene product in infected hosts (see, e.g. Logan & Shenk (1984) Proc. Natl. Acad. Sci. USA 81, 3655-3659). Specific initiation signals may also be required for efficient translation of inserted MRGX2 nucleotide sequences. These signals include the ATG initiation codon and adjacent sequences. In cases where an entire MRGX2 gene or cDNA, including its own initiation codon and adjacent sequences, is inserted into the appropriate expression vector, no additional translational control signals may be needed. However, in cases where only a portion of the MRGX2 coding sequence is inserted, exogenous translational control signals, including, perhaps, the ATG initiation codon, must be provided. Furthermore, the initiation codon must be in frame with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc (see Bittner et al. (1987) Methods in Enzymol. 153, 516-544).

In addition, a host cell strain may be chosen which modulates the expression of the inserted sequences, or modifies and processes the gene product in the specific fashion desired. Such modifications (e.g. glycosylation) and processing (e.g. cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. Accordingly, eukaryotic host cells which possess the cellular machinery for proper processing of the primary transcript, glycosylation, and phosphorylation of the gene product may be used. Such mammalian host cells include, but are not limited to, CHO, Vero, BHK, HeLa, COS, MDCK, HEK293, 3T3 and WI38 cell lines.

For long-term, high-yield production of recombinant proteins, stable expression is preferred. For example, cell lines which stably express the MRGX2 sequences described above may be engineered. Rather than using expression vectors which contain viral origins of replication, host cells can be transformed with DNA controlled by appropriate expression control elements (e.g. promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the MRGX2 gene product. Such engineered cell lines may be particularly useful in screening and evaluation of compounds that affect the endogenous activity of the MRGX2 gene product.

A number of selection systems may be used, including but not limited to the herpes simplex virus thymidine kinase (Wigler et al. (1977) Cell 11, 223), hypoxanthine-guanine phosphoribosyltransferase (Szybalska & Szybalski (1962) Proc. Natl. Acad. Sci. USA 48, 2026), and adenine phosphoribosyltransferase (Lowy et al. (1980) Cell 22, 817) genes can be employed in tk⁻, hgprt⁻ or aprt⁻ cells, respectively. Also, antimetabolite resistance can be used as the basis of selection for the following genes: dhfr, which confers resistance to methotrexate (Wigler et al. (1980) Natl. Acad. Sci. USA 77, 3567; O'Hare, et al. (1981) Proc. Natl. Acad. Sci. USA 78, 1527); gpt, which confers resistance to mycophenolic acid (Mulligan & Berg (1981) Proc. Natl. Acad. Sci. USA 78, 2072); neo, which confers resistance to the aminoglycoside G-418 (Colberre-Garapin et al. (1981) J. Mol. Biol. 150:1); and hygro, which confers resistance to hygromycin (Santerre et al. (1984) Gene 30, 147).

MRGX2 expression in mammalian cells can also be achieved by upregulating the expression of MRGX2. This can be achieved, for example, by infection of B-lymphocytes or B-lymphocyte-derived cell lines with Epstein Barr Virus (see Birkenbach, M.P. et al (1993), J. Virol. 67, 2209-2220), or other substances may be identified that also upregulate MRGX2 expression by using methods of the invention. Upregulation of MRGX2 expression can also be achieved by recombinant means, for example as described in Patent EP 411,678 for human erythropoietin. Other methods are available, such as the system developed by Athersys and described, for example, in patent applications WO 99/15650 or WO 00/49162.

Antibodies that specifically recognize one or more epitopes of MRGX2, or epitopes of conserved variants of MRGX2, or peptide fragments of MRGX2 are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of MRGX2 in a biological sample and may, therefore, be utilized as part of a diagnostic or prognostic technique whereby patients may be tested for abnormal amounts of MRGX2. Antibodies that specifically recognize mutant forms of MRGX2, may be particularly useful as part of a diagnostic or prognostic technique. Such antibodies may also be utilized in conjunction with, for example, compound screening schemes, as described, above, for the evaluation of the effect of test compounds on expression and/or activity of the MRGX2 gene product. Additionally, such antibodies can be used in conjunction with the gene therapy techniques, e.g. to evaluate the normal and/or engineered MRGX2-expressing cells prior to their introduction into the patient. Such antibodies may additionally be used as a method for the inhibition of abnormal MRGX2 activity. Thus, such antibodies may, therefore, be utilized as part of neuropeptide-related disorder treatment methods.

For the production of antibodies, various host animals may be immunized by injection with MRGX2, a MRGX2 peptide (e.g. one corresponding to extracellular loops or the extracellular domain), truncated MRGX2 polypeptides (MRGX2 in which one or more domains, e.g. the transmembrane domain or cellular domain, has been deleted), functional equivalents of MRGX2 or mutants of MRGX2. Such host animals may include but are not limited to rabbits, mice, hamsters and rats, to name but a few. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum.*

Polyclonal antibodies are heterogeneous populations of antibody molecules derived from the sera of the immunized animals.

Monoclonal antibodies, which are homogeneous populations of antibodies to a particular antigen, may be obtained by any technique which provides for the production of antibody molecules by continuous cell lines in culture. These include, but are not limited to, the hybridoma technique of Kohler and Milstein, ((1975) Nature 256, 495-497 and U.S. Patent No. 4,376,110), the human B-cell hybridoma technique (Kosbor et al. (1983) Immunology Today 4, 72; Cole et al. (1983) Proc. Natl. Acad. Sci. USA 80, 2026-2030), and the EBV-hybridoma technique (Cole et al. (1985) Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

In addition, techniques developed for the production of "chimeric antibodies" (Morrison et al. (1984) Proc. Natl. Acad. Sci., 81, 6851-6855; Neuberger et al. (1984) Nature, 312, 604-608; Takeda et al. (1985) Nature, 314, 452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region.

Alternatively, techniques described for the production of single chain antibodies (U.S. Patent 4,946,778; Bird (1988) Science 242, 423-426; Huston et al. (1988) Proc. Natl. Acad. Sci. USA 85, 5879-5883; and Ward et al. (1989) Nature 334, 544-546) can be adapted to produce single chain antibodies against MRGX2 gene products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments or by papain digestion of antibody molecules. Alternatively, Fab expression libraries may be constructed (Huse et al. (1989) Science, 246, 1275-1281) to allow rapid and easy identification of monoclonal Fab fragments with the desired specificity.

Antibodies to MRGX2 can, in turn, be utilized to generate anti-idiotype antibodies that "mimic" MRGX2, using techniques well known to those skilled in the art (see, e.g. Greenspan & Bona (1993) FASEB J 7, 437-444; and Nissinoff (1991) J. Immunol. 147, 2429-2438). For example antibodies which bind to the MRGX2 extracellular domain and competitively inhibit the binding of neuropeptides to MRGX2 can be used to generate anti-idiotypes that "mimic" the extracellular domain and, therefore, bind and neutralize neuropeptides. Such neutralizing anti-idiotypes or Fab fragments of such anti-idiotypes can be used in therapeutic regimens to neutralize the native ligand and treat neuropeptide-related disorders, such as all types of sleep disorders, pain, stroke, memory disorders, diabetes, cancer, obesity, cardiovascular disorders, depression, sexual dysfunction, urinary tract and bladder disorders, anti-infective treatments, gastrointestinal disorders, and other disorders.

Alternatively, antibodies to MRGX2 that can act as agonists of MRGX2 activity can be generated. Such antibodies will bind to the MRGX2 and activate the signal transducing activity of the receptor. In addition, antibodies that act as antagonist of MRGX2 activity, i.e. inhibit the activation of MRGX2 receptor would be particularly useful for treating neuropeptide- related disorders, such as all types of sleep disorders, pain, stroke, memory disorders, diabetes, cancer, obesity, cardiovascular disorders, depression, sexual dysfunction, urinary tract and bladder disorders, anti-infective treatments, gastrointestinal disorders, and other disorders.

Genetically engineered cells that express soluble MRGX2 extracellular domains or fusion proteins e.g. fusion Ig molecules can be administered *in vivo* where they may function as "bioreactors" that deliver a supply of the soluble molecules. Such soluble MRGX2 polypeptides and fusion proteins, when expressed at appropriate concentrations, should neutralize or "mop up" the native ligand for MRGX2, and thus act as inhibitors of MRGX2 activity and may therefore be used to treat neuropeptide-related disorders, such as all types of sleep disorders, pain, stroke, memory disorders, diabetes, cancer, obesity, cardiovascular disorders, depression, sexual dysfunction, urinary tract and bladder disorders, anti-infective treatments, gastrointestinal disorders, and other disorders.

The compounds of the invention, i.e. modulators of MRGX2 receptor, can be used to treat conditions such as, for example, all types of sleep disorders, pain, stroke, memory disorders, diabetes, cancer, obesity, cardiovascular disorders, depression, sexual dysfunction, urinary tract and bladder disorders, anti-infective treatments, gastrointestinal disorders, and other disorders.

Toxicity and therapeutic efficacy of such compounds can be determined by standard pharmaceutical procedures in cell cultures or experimental animals, e.g. for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e. the concentration of the test compound which achieves a half-maximal inhibition of symptoms) as determined in cell culture. Such information can be used to more accurately determine useful doses in humans. Levels in plasma may be measured, for example, by high performance liquid chromatography.

Pharmaceutical compositions for use in accordance with the present invention may be formulated in conventional manner using one or more physiologically acceptable carriers or excipients.

Thus, the compounds and their physiologically acceptable salts and solvates may be formulated for administration by inhalation or insufflation (either through the mouth or the nose) or oral, buccal, parenteral or rectal administration.

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycolate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compounds may be formulated for parenteral administration by injection, e.g. by bolus injection or continuous infusion. Formulations for injection may be presented in unit dosage form, e.g. in ampules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g. sterile pyrogen-free water, before use.

The compounds may also be formulated in rectal compositions such as suppositories or retention enemas, e.g. containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

### Examples

The examples below are carried out using standard laboratory procedures, well known to a person skilled in the art, but reference may be made in particular to Sambrook *et al.,* Molecular Cloning, A Laboratory Manual (2001) and Ausubel *et al.,* Short Protocols in Molecular Biology (1999) 4^{th} Ed, John Wiley & Sons, Inc. PCR is described in US-A-4683195, US-A-4800195 and US-A-4965188.

The examples are intended to illustrate but not limit the invention. While they are typical of the methods that might be used, other procedures known to those skilled in the art may also be used. References are made to the following sequence listings and figures:
SEQ ID NO: 1: cDNA sequence of receptor MRGX2.
SEQ ID NO: 2: Peptide sequence of receptor MRGX2.
SEQ ID NO: 3: Forward primer for cloning receptor MRGX2 cDNA
SEQ ID NO: 4: Reverse primer for cloning receptor MRGX2 cDNA
SEQ ID NO: 5: Forward primer for tissue distribution studies for MRGX2
SEQ ID NO: 6: Reverse primer for tissue distribution studies for MRGX2
Figure 1: Dose response curve for MRGX2 for several neuropeptides
Figure 2: Dose response curve for MRGX2 for several neuropeptides

### Example 1: Bioinformatics analysis of receptor MRGX2

A bioinformatic analysis was performed on the sequence of MRGX2.

### (a) Blast against protein databases

The sequence of receptor MRGX2 was searched against Swissprot using the BLAST algorithm (Basic Local Alignment Search Tool (Altschul SF (1993) J.Mol. Evol. 36, 290-300; Altschul, SF et al (1990) J. Mol. Biol. 215, 403-410; Altschul SF et al (1997) Nucl. Acids Res. 25, 3389-3402)) to identify the closest protein match. In this case the top hit was to:
P12526: Rat MAS protooncogene, a member of the GPCR family of unknown function.
These results confirm that receptor MRGX2 is a member of the GPCR family.

### (b) BLAST search against a non-redundant human GPCR database

Receptor MRGX2 was searched against a non-redundant human GPCR database comprising mainly sequences from Genbank and the Derwent Geneseq database (containing sequences from patent applications and patents) in order to identify the class of agonist for this receptor. The top ten hits are shown below:

| **Full Name** | **%ID** | **Fraction*** | **Evalue*** |
|---|---|---|---|
| Dorsal root receptor 4 DRR4 (geneseqp) | 65% | 215/329 | e-115 |
| Dorsal root receptor 3 DRR3 (geneseqp) | 64% | 212/329 | e-113 |
| Dorsal root receptor 6 DRR6 (geneseqp) | 62% | 205/329 | e-107 |
| Dorsal root receptor 1 DRR1 (geneseqp) | 62% | 205/329 | e-106 |
| Dorsal root receptor 5 DRR5 (geneseqp) | 62% | 204/325 | e-105 |
| Dorsal root receptor 2 DRR2 (geneseqp) | 61% | 201/329 | e-103 |
| G-protein coupled receptor R97222 (geneseqp) | 37% | 107/286 | 2e-43 |
| MAS proto-oncogene | 36% | 113/312 | 2e-43 |
| MAS-related G protein-coupled receptor MRG. | 35% | 99/282 | 7e-39 |
| Thoracic aorta G-protein coupled receptor (geneseqp) (fragment) | 35% | 99/282 | 3e-38 |

### Example 2: Tissue distribution of the mRNA encoding receptor MRGX2 by polymerase chain reaction (PCR)

The primers used were:

### Forward Primer:

5'-GCACCAGTGGAGGTTTTCTGAGC-3' (SEQ ID NO: 5)

### Reverse Primer:

5'-TCATCACAGTGGTGAAGAAGC-3' (SEQ ID NO: 6)
PCR conditions: 94°C - 1 minute. Then 30 cycles of 94°C - 30 seconds, 55°C - 30 seconds, 72°C - 1 minute. Final cycle = 72°C - 10 minutes.

As template, commercially available cDNA was used from various tissues. The receptor was found to be expressed in dorsal root ganglion, vascular smooth muscle, brain, liver, heart, pancreas, thymus, leukocytes and testis, whereas it was not found by this method in lung, skeletal muscle, kidney, spleen, prostate, ovary, small intestine, colon, and placenta.

### Example 3: Cloning and transient expression of receptor MRGX2 in mammalian cell lines

The cDNA encoding receptor MRGX2 was obtained by polymerase chain reaction (PCR) using brain cDNA as template. The PCR primers were designed around the ATG start codon and the stop codon, leading to the amplification of the coding sequence only, eliminating any 5' or 3' untranslated regions. The primers used were as shown in SEQ ID NOs 3 and 4:
MRGX2 forward primer (SEQ ID No: 3)
5'-ACC ATG GAT CCA ACC ACC CCG GCC-3'
MRGX2 reverse primer (SEQ ID No: 4)
5'-CTA CAC CAG ACT GCT TCT CGA CAT C-3'

### PCR mix:

| | |
|---|---|
| MRGX2 primers | 1 µl (10 µM stock) |
| Human brain cDNA | 2 µl (400ng) |
| dNTPs (concentration as per kit) | 1 µl |
| platinum Taq high fidelity Polymerase (LTI, Inc.) | 0.5 µl |
| 10x amplification Buffer (from PCR kit) | 5 µl |
| MgSO₄ | 1.5 µl |
| dH₂O | 39 µl |

### PCR cycle:

(1) 94 °C 2 mins
(2) 94°C 30 seconds
(3) 54°C 30 seconds
(4) 68°C 2 mins
   Steps (2) through to (4) were repeated for a further 27 cycles.
(5) 68°C 15 mins
(6) 4°C soak.

The PCR product was then inserted into a suitable mammalian cell expression vector, e.g. into pcDNA3.1/V5-His-TOPO (Invitrogen), using the manufacturer's protocols. The cDNA insert could easily be excised from the pcDNA3.1/V5-His-TOPO vector, e.g. using restriction sites *KpnI* and *NotI,* and ligated into a different expression vector, e.g. pcDNA4HISmaxB digested with the same enzymes, using standard molecular biology techniques (e.g. according to Sambrook, *et al.,* eds. (1989) Molecular Cloning: A Laboratory Manual, Cold Spring Harbor Laboratory Press, New York, NY, USA) and using T4 DNA ligase to join the fragments. The vector pcDNA4HISmaxB is used as it contains elements that up-regulate the level of gene transcription over standard pcDNA3.1 vectors.

5 x 10⁶ cells of a suitable host cell line, chosen to express a low number of endogenous receptors (e.g. HEK293 or CHO-K1) and engineered to stably express G_{α15} / G_{qi-5,} were transiently transfected with 7.5 µg of cDNA encoding receptor MRGX2 in a suitable mammalian cell expression vector, or vector alone, using Lipofectamine Plus® reagent (Gibco BRL) as per the manufacturer's protocol. If the cells were intended for a ligand binding assay or microphysiometry, they were incubated for 24-72 hours post transfection, and then harvested. If a microtitre plate-based assay was intended, the cells were detached from the flask using Trypsin/EDTA solution (LTI) 24 hours post transfection, and seeded into e.g. a black sided, Poly-D-lysine-treated, 96-well plate (Becton Dickinson) at 5 x 10⁴ cells/well density. The plates were left overnight to allow the cells to adhere to the bottom of the wells.

### Example 4: Engineering of stable cell lines expressing high levels of receptor MRGX2

A suitable host cell line, e.g. HEK293 cells or CHO cells (potentially engineered to express a desired G protein), is transfected as described in Example 3, with a suitable mammalian cell expression vector containing the cDNA (preferably without any 5' or 3' untranslated regions) encoding receptor MRGX2, and containing a selectable marker, e.g. a neomycin resistance gene. Following transfection, selection pressure is applied, e.g. by adding 400-800 µg/ml G418 to the growth medium and thereby killing all cells which have not taken up the vector which contains the neomycin resistance gene. After about 3-4 weeks of selection, individual clones are picked and expanded for further analysis. The individual clones can be analysed e.g. by Northern blot, using a labelled probe designed from the receptor MRGX2 cDNA sequence.

### Example 5: Ligand binding assays

Cells expressing receptor MRGX2, either 24-72 hours after transient transfection as described in Example 3, or engineered as described in Example 4, are harvested by scraping, resuspended in 20 ml of ice-cold assay buffer (50 mM Tris-HCl pH 7.4), homogenised, and the resulting suspension is centrifuged at 20,000g, 4°C for 30 minutes. The supernatant is decanted, the pellet resuspended in 3 ml of assay buffer and re-homogenised (50 mM Tris-HCl pH7.4). The protein concentration is determined via Bradford's assay (Biorad), according to the manufacturer's recommendations.

Aliquots of this membrane preparation containing 200 µg protein are then incubated with various potential ligands, radiolabeled to high specific activity, for about 2 hrs at room temperature or at 30°C (the optimal conditions, ion concentrations, incubation time and temperature need to be determined for each ligand). To terminate incubations, samples are rapidly filtered using the Brandell cell harvester onto Wallac Filtermats (Perkin Elmer) (which have been previously soaked (for 1h) in a 0.3% (v/v) solution of PEI (polyethylenimine; Sigma) in assay buffer to reduce Filtermat binding). Immediately, the Filtermat/wells are washed four times in rapid succession with 2 ml of assay buffer per well. Filtermats are dried using a microwave oven, and Meltilex scintillant (Perkin Elmer) is melted onto the Filtermats using the Wallac Meltilex heat sealer. The bound radioactivity on the Filtermats is determined using the Wallac betaplate scintillation counter.

The specific binding is defined as the difference between total radioactivity bound minus the radioactivity measured in the presence of an excess of unlabelled ligand. Mock-transfected cells are also measured to assess whether the host cells express receptors for the ligands used endogenously.

### Example 6: Functional assay measuring intracellular calcium mobilisation

Fluorescence Imaging Plate Reader (FLIPR®) technology was employed as a means to detect activation of receptor MRGX2 by various potential GPCR agonists.

Cells were transfected as described in Example 3. 24 hrs post-transfection, the cells were detached from the flask using Trypsin/EDTA solution (LTI) and seeded into a black sided, Poly-D-lysine-treated, 96-well plate (Becton Dickinson) at 5 x 10⁴ cells/well density. The plates were left overnight to allow the cells to adhere to the bottom of the wells. The medium was removed from the cells and replaced with 100 µl warm (37°C) dye loading solution (50 µg Fluo3 (Molecular Probes) in 20 µl DMSO + 20% pluronic acid in DMSO, added to 11 ml Dulbecco's Modified Eagles Medium containing 1x Probenecid (100x Probenecid - 0.71 g Probenecid was dissolved in 5 ml 1M NaOH and 5 ml Dulbeccos' Phosphate Buffered Saline (PBS), per plate; Probenecid (Molecular Probes) inhibits activity of the anion transport protein, thus improving dye loading). The plates were then incubated for 1 hr at 37°C. Plates were subsequently washed with 250 µl of wash buffer per well (5 ml 100x Probenecid stock + 495 ml PBS, pH 7.4) 4 times. The plates were returned to the 37°C/5%CO₂ incubator for 30 mins prior to processing within the FLIPR® instrument. The FLIPR® processing involved reading the fluorescence for all samples for 2 minutes; during this time the fluorescence baseline was determined for 10 seconds. The desired amount of compounds (i.e. potential GPCR agonists) was then automatically transferred to the wells and the fluorescence was continuously monitored for the remainder of the time. All compounds were diluted in wash buffer.

Compounds capable of acting as agonists for the receptor were identified by them causing a transient rise in fluorescence, and therefore in intracellular calcium, in the cells.

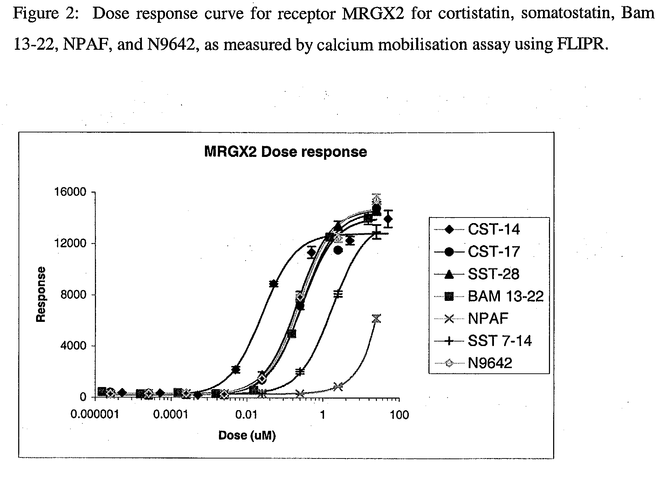

Dose response curves of the neuropeptides giving the best responses are shown in Figure 1 and Figure 2. Preliminary EC50 data are shown in Table 1:

**Table 1:**

| Preliminary EC50 data for receptor MRGX2: | | |
|---|---|---|
| **Peptide** | **EC**_{**50**} **(µM)** | **Standard deviation** |
| Cortistatin 14 | 0.025 | 0.003 |
| Cortistatin 17 | 0.099 | 0.012 |
| BAM 13-22 | 0.100 | 0.028 |
| Somatostatin 3-14 | 0.160 | 0.034 |
| D-Trp-Somatostatin | 0.179 | 0.039 |
| Somatostatin 28 | 0.218 | 0.020 |
| N9642 (β-(2-Naphthyl)-D-Ala-Cys-Tyr-D-Trp-Lys-Val-Cys-Thr amide [Disulfide Bridge: 2-7] (sst₂R agonist)) | 0.251 | 0.023 |
| HS024 (Acetyl-Cys³,Nle⁴,Arg⁵,D-2-Nal⁷,Cys¹¹)-a-MSH (3-11) amide | 0.420 | 0.179 |
| BAM 22 | 0.420 | 0.139 |
| NPFF | 0.430 | 0.143 |
| Somatostatin 3-10 | 0.540 | 0.134 |
| EP1 | 0.750 | 0.088 |
| Somatostatin 14 | 0.780 | 0.052 |
| Oxytocin | 1.470 | 0.129 |
| Cyclosomatostatin | 1.470 | 0.242 |
| Somatostatin 7-14 | 1.810 | 0.211 |
| Dynorphin A | 1.850 | 0.837 |
| [Arg⁸] vasopressin | 2.020 | 0.179 |
| Somatostatin 2-9 | 2.270 | 0.456 |
| Substance P | 2.470 | 0.717 |
| (Ser⁴, Ile⁸)-Oxytocin | 7.400 | 1.91 |
| BAM 8-22 | 8.500 | 1.100 |
| Hexarelin | 9.500 | |
| (Deamino-Cysl,D-Orn⁸) Vasopressin | > 10 | |
| Octreotide | > 10 | |
| EP2 | > 10 | |
| EP3 | > 10 | |
| NPAF | > 10 | |
| NPVF | > 10 | |
| NPSF | > 10 | |
| Nociceptin | > 10 | |
| HS014 | > 10 | |
| Apelin 13 | > 10 | |
| Adenine | No response | |

### Example 7: Functional assay using microphysiometry

Agonism at most receptors will lead to the cells requiring extra energy to respond to the stimulus. In order to generate the energy, the metabolic activity will increase, leading to a small but measurable acidification of the surrounding medium. This small pH change can be measured using the Cytosensor microphysiometer developed by Molecular Devices Corporation.

Cells expressing receptor MRGX2, produced as described in Example 3 or 4, are harvested gently with non-enzymatic cell dissociation fluid (Sigma), washed with a physiological buffer, and seeded in Cytosensor assay cups in a medium with low buffer capacity and allowed to adhere as a monolayer; alternatively, a high cell density can be achieved by using the agarose entrapment method as described in the manufacturer's protocols. The cups are then placed in the Cytosensor and equilibrated for 1 hour by gently pumping low-buffer medium over the cells. Then, low-buffer medium containing the desired amount of potential agonist is gently pumped over the cells, and the acidification rate of the cells continued to be monitored. The agonist for the expressed receptor is identified as the substance that increased the metabolic rate in transfected cells, but not the respective host cell line.

### Example 8: Screening of tissue extracts and body fluids

The microphysiometry assay of Example 7 can also be used to identify agonists in tissue extracts and body fluids. Instead of passing isolated substances over the cells as in Example 7, tissue extracts or body fluids can be used; if a response is obtained, the extract or fluid can then be fractionated using column chromatography or other separation techniques known in the art to identify, usually in multiple cycles of separation and testing of fractions, the substance which caused the effect. This way, previously unknown natural agonists for orphan receptors can be found.

### Example 9: CRE-Luciferase assay

HEK293 cells are cultured to ∼80% confluence at which time they are co-transfected with plasmids pCRE-Luc (CRE₄-Luciferase, Stratagene) and a plasmid construct containing the orphan GPCR using the lipid transfection reagent LIPOFECTAMINE Plus⁽™⁾ (Invitrogen) as recommended by the manufacturer. Cells are cultured for 24 hours post-transfection before being washed with phosphate buffered saline (pH 7.4), recovered in Dulbecco's modified Eagle medium (supplemented with 10% (v/v) foetal calf serum) and seeded as 100µl aliquots (5x10⁴ cells) into white 96 well tissue culture plates. Following a further 24 hour incubation, compounds are added in a 20µl volume to triplet wells, and plates are left to incubate for a minimum of 5 hours. The growth medium is then removed and the cells are lysed in Glo lysis buffer (Promega) followed by the addition of 100µl of SteadyGlo luciferase detection reagent (Promega). The level of expressed luciferase is quantified on a Tecan Ultra(™) Reader (1 second per well). Where experiments are designed to measure drug-mediated dose-response effects a control dose-response is always included. Data obtained for compounds are normalised relative to the control values determined in that experiment. All experiments are performed on three or more separate occasions, and mean values±S.E.M. determined. IC₅₀ values and apparent efficacies are calculated by fitting a four-parameter logistic curve (Labstats software, Microsoft Excel) through the mean normalised data. For antagonists, p*A*₂ values are determined by performing Schild analysis (Arunlakshana, O. and Schild, H.O. (1959) Brit. J. Pharmacol. 14, 48-58).

### Example 10: β-lactamase assay

A CHO cell line engineered to stably contain cyclic AMP response elements (CRE) functionally linked to the coding region of reporter gene β-lactamase as well as the nuclear factor of activated T-cell promoter NF-AT (Flanagan et al (1991) Nature 352, 803-807) linked to the coding region of reporter gene β-lactamase (CHO-CRE-NFAT-BLA) is transfected stably as described in Example 4, with a plasmid containing the cDNA encoding receptor MRGX2 functionally linked to a promoter that drives expression in mammalian cells, e.g. pcDNA3.1, and selected for stable expression of receptor MRGX2.

The CHO-CRE-NFAT-BLA cells expressing receptor MRGX2 are then seeded at 4x 10³ cells per well in 96-well plates, and incubated for 60 hours at 37°C in a CO₂ incubator (5% CO₂). The medium is then removed, and 90 µl starvation medium (DMEM with high glucose, 0.1mM Non-essential amino acids, 1mM sodium pyruvate, 25mM Hepes buffer, without serum or antibiotics) is added to each well, and the cells are incubated overnight. The cells are then stimulated by addition of 10 µl neuropeptide ligand such as cortistatin, somatostatin, α-MSH, Bam22, dynorphin A, substance P, or neuropeptide FF (or 1 µM ionomycin for positive control) prepared in DMEM with 1% dialysed fetal bovine serum per well. Following incubation at 37°C/5% CO₂ for 5 hours, 20µl of 6x dye solution (CCF2 Loading kit from Aurora, Cat # 00 100 012, contains solutions A-D; to prepare 6x dye solution, 36µl solution A (CCF2-AM), 180µl solution B, 2.8ml solution C and 225µl solution D are mixed according to the instructions) are added per well, and the plate is incubated on a rocking platform in the dark at room temperature for 1 hour (rocking at 40 cycles per minute). The fluorescence is then measured in a Cytofluor 4000 (PerSeptive Biosystems), using an excitation wavelength of 405 nm, and measuring emission at wavelengths of 450 nm and 530 nm.

When the ligand stimulates the receptor and the response leads to either a change in cAMP concentration or in calcium concentration in the cells, β-lactamase will be expressed in the cells. The dye is composed of a blue (coumarin) and a green (fluorescein) component which are linked by a β-lactam linker group. When excited at 405 nm, fluorescence energy transfer will occur within the uncleaved molecule, and the emission wavelength will be green (around 530 nm). When the linker is cleaved by β-lactamase, no energy transfer can occur, and blue fluorescence results, measured at 450 nm. Measuring the ratio of blue to green fluorescence will give an indication of receptor stimulation. The ratio is agonist dose dependent, and can be used to rank agonists for the receptor.

## Claims

1. Use of cortistatin, somatostatin, Bam 13-22, α-MSH, neuropeptide FF, dynorphin A or substance P or an analogue or mimetic of any one of these neuropeptides as a ligand for receptor MRGX2.

2. Use of cortistatin, somatostatin, Bam 13-22, α-MSH, neuropeptide FF, dynorphin A or substance P or an analogue or mimetic of any one of these neuropeptides as a modulator for receptor MRGX2.

3. Use of cortistatin, somatostatin, Bam 13-22, α-MSH, neuropeptide FF, dynorphin A or substance P or an analogue or mimetic of any one of these neuropeptides to elicit a functional response on receptor MRGX2.

4. Use of any one of claims 1 to 3, wherein the use is of cortistatin or of somatostatin or of Bam 13-22 or of α-MSH.

5. A method of screening for compounds that are modulators of receptor MRGX2, using cortistatin, somatostatin, Bam 13-22, α-MSH, neuropeptide FF, dynorphin A or substance P or an analogue or mimetic of any one of these neuropeptides as ligand.

6. A method of screening for compounds that are modulators of a novel neuropeptide receptor, including but not limited to the following steps:
(a) contacting a sample of receptor MRGX2 with a ligand selected from cortistatin, somatostatin, Bam 13-22, α-MSH, neuropeptide FF, dynorphin A or substance P or an analogue or mimetic of any one of these neuropeptides;
(b) contacting a similar sample of receptor MRGX2 or membranes prepared from said cells with both the ligand used in step (a) and a test compound or mixture of test compounds;
(c) comparing the results of (a) and (b) to determine whether the binding of the ligand used is affected by the presence of the test compound or mixture of test compounds.

7. A method of screening for compounds that are modulators of a novel neuropeptide receptor, including but not limited to the following steps:
(a) contacting a sample of receptor MRGX2 with a ligand selected from cortistatin, somatostatin, Bam 13-22, α-MSH, neuropeptide FF, dynorphin A or substance P or an analogue or mimetic of any one of these neuropeptides, with a detectable label attached;
(b) contacting a similar sample of receptor MRGX2 or membranes prepared from said cells with both the labelled ligand used in step (a) and a test compound or mixture of test compounds;
(c) comparing the amount of label bound in (a) and (b) to determine whether the binding of the ligand used is affected by the presence of the test compound or mixture of test compounds.

8. A method of expressing a novel neuropeptide receptor, comprising transferring a suitable expression vector comprising SEQ ID NO: 1 or variants or homologues thereof, into suitable host cells, and culturing said host cells under conditions suitable for the expression of the receptor.

9. The method of claim 8, wherein the host cells are mammalian cells or insect cells.

10. A method of expressing a novel neuropeptide receptor, comprising upregulating the expression of MRGX2 in a suitable cell.

11. The method of claim 6 or claim 7, wherein the sample of MRGX2 comprises cells prepared by the method of any one of claims 8, 9, or 10, cells naturally expressing MRGX2, or membranes prepared from said cells, or MRGX2 protein enriched or purified from said cells or membranes.

12. A method for screening for agonists of a novel neuropeptide receptor, comprising the steps:
(a) adding a test compound or a mixture of test compounds to suitable cells expressing MRGX2,
(b) measuring whether a functional response is seen.

13. The method of claim 10, wherein the functional response is a transient rise in intracellular calcium concentration.

14. The method of claim 10, wherein the functional response is acidification of the surrounding medium as measured by microphysiometry.

15. The method of claim 10, wherein the functional response is activation of a reporter gene linked to a cyclic AMP response element.

16. A method for screening for antagonists of a novel neuropeptide receptor, comprising the steps:
(a) adding a test compound or a mixture of test compounds to suitable cells expressing MRGX2,
(b) adding cortistatin, somatostatin, Bam 13-22, α-MSH, neuropeptide FF, dynorphin A, substance P, or an analogue or mimetic of any one of these neuropeptides, or an agonist as identified by the methods of any one of claims 12 to 15;
(c) measuring whether a functional response is seen, identifying antagonists as the test compounds which reduce the functional response to the agonist.

17. The method of any one of claims 12 to 16, wherein suitable cells expressing MRGX2 are cells naturally expressing MRGX2, or cells produced by the method of any one of claims 8, 9, or 10.

18. The compound identified by the method of any one of claims 5 to 7 or 12 to 17.

19. A pharmaceutical composition comprising a compound identified using the methods of any one of claims 5 to 7 or 12 to 17. and a suitable pharmaceutically acceptable carrier.

20. A method of preparing a pharmaceutical composition which comprises determining whether a compound is a novel neuropeptide receptor agonist or antagonist using the method of any one of claims 5 to 7 or 12 to 17., and admixing said compound with a pharmaceutically acceptable carrier.

21. A method of diagnosing a novel neuropeptide-receptor-mediated disorder in a mammal, comprising
(a) measuring the level of MRGX2 gene expression, or
(b) measuring the neuropeptide-dependent activity of MRGX2 in a patient sample, and comparing said measurement to that determined from clinically normal individuals.
